# EUROPEAN PATENT APPLICATION

(11) **EP 4 491 118 A1**
(43) Date of publication of application: **15.01.2025**
(21) Application number: 24188385.9
(22) Date of filing: 12.07.2024
(51) Int. Cl.: A61B 5/15, B01L 3/00, A61B 5/151

(54) **COLLECTOR ACCESSORY FOR SMALL VOLUME COLLECTION CONTAINERS AND RELATED SAMPLE COLLECTION METHODS**

(30) Priority: 13.07.2023 US 202363513362 P
(71) Applicant: Becton, Dickinson and Company, Franklin Lakes, NJ 07417 (US)
(72) Inventor: LOSADA, Robert J., Astoria, NY 11105 (US); WIGH, Shruti, Maywood, NJ 07607 (US)
(74) Representative: dompatent von Kreisler Selting Werner - Partnerschaft von Patent- und Rechtsanwälten mbB

(57) **Abstract**

A sample collection accessory device configured for attachment to an open end of a collection container includes a cover and a collector. The cover includes an inner portion configured for insertion into the collection container having a sidewall having an inner surface defining a channel through the accessory device and an outer surface with an outer diameter sized to seal against an inner surface of a sidewall of the container. The cover also includes an outer portion extending radially outward from the inner portion configured to extend over an end of the sidewall of the collection container. The collector extends distally from the cover and is configured for directing a biological sample to the channel defined by the inner surface of the inner portion of the cover. A method for automated blood sample processing using a collection container and an accessory device is also provided.

## Description

### CROSS-REFERENCE TO RELATED APPLICATION

The present application claims priority to United States Provisional Application No. 63/513,362 entitled "Collector Accessory for Small Volume Collection Containers and Related Sample Collection Methods" filed July 13, 2023, the disclosure of which is hereby incorporated by reference in its entirety.

### BACKGROUND OF THE INVENTION

### Field of the Invention

The present disclosure relates to a collector accessory or accessory device, such as a cap, closure, cover, lid, sealing member, or similar device, configured to be mounted to an open end of a small volume collection container, such as a small volume collection tube configured for receiving a capillary blood sample, including a collector for directing a biological sample obtained from a patient, such as a sample used for medical diagnostic testing, through the open end and into an interior of the small volume collection container.

### Description of Related Art

Devices for biological sample collection are commonly used in the medical industry. One common blood collection technique is capillary blood collection for obtaining a small volume blood sample, which can be used for testing for treatment and monitoring of a variety of conditions and diseases. For example, capillary blood samples are regularly collected for diabetes patients in order to perform blood testing to monitor the patient's blood sugar levels and/or other physiological markers. Test kits, such as cholesterol test kits, also often require a user to obtain a capillary blood sample for analysis.

Blood collection processes for obtaining a capillary blood sample usually involve pricking a finger or other suitable body part of the patient and allowing the blood sample to collect in a small volume container. Different types of lancet devices have been developed for puncturing the patient's skin to obtain the capillary blood sample. Typically the amount of blood needed for such tests is relatively small and a small puncture# wound or incision by the lancet device normally provides a sufficient amount of blood for these tests. The small volume collection container used for capillary blood collection is often a tubular container, such as a straight-walled micro-tube. After the sample is collected into the container, the collection container can be sealed by a cap assembly inserted into or over the open end of the collection container. The sealed container can be provided to a laboratory for processing, such as for automated diagnostic testing.

Automated testing processes can include using sorting machines to sort collection containers (e.g., by type, size, and/or contents). Other automated processes can include filling selected specimen collection containers with specific additives, centrifugation, vision-based specimen quality analysis, serum level analysis, decapping, aliquoting, and/or automated labeling of containers. Conventional collection containers may not be compatible with any or all automated processing machines used for processing and analysis of collected blood samples. For example, automated sorting machines may not be able to sort conventional specimen collection containers (e.g., by type, size, and/or contents) due to a unique or irregular geometry of components of some collection containers, lids, closures, seals, or other components. Therefore, there is a need in the art for more universal sample collection containers having a regular geometry compatible for use with a wide variety of automated processing machines. The sample collection containers and assemblies disclosed herein are configured to provide such universal compatibility meaning that the sample collection containers can be used with many different automated machines and devices.

### SUMMARY OF THE INVENTION

In view of the difficulties in processing conventional sample collection containers using automated systems and devices, the present disclosure is directed to sample collection containers having simple geometries that are compatible with a wide variety of automated devices and systems. In particular, the sample collection containers disclosed herein are desirably compatible with a wide variety of front end automation devices, automated analyzers, and/or back end automation devices, thereby providing for improved workflow efficiencies over conventional manual processing. The disclosure is also directed to accessory devices or accessories for such blood collection containers, which can be used during blood collection, and removed prior to automated processing of collection containers containing a biological sample. Accordingly, the accessories do not interfere with automated processing devices, such as sorting devices, additive injection devices, centrifuge devices, or quality analysis devices used for processing biological samples.

According to an aspect of the present disclosure, a sample collection accessory device configured for attachment to an open end of a collection container includes a cover and a collector. The cover includes an inner portion configured for insertion into the collection container having a sidewall having an inner surface defining a channel through the accessory device and an outer surface with an outer diameter sized to seal against an inner surface of a sidewall of the container. The cover also includes an outer portion extending radially outward from the inner portion configured to extend over an end of the sidewall of the collection container. The collector extends distally from the cover and is configured for directing a biological sample to the channel defined by the inner surface of the inner portion of the cover.

According to another aspect of the present disclosure, a collection assembly includes a collection container having an open first end, a second end, and an annular sidewall extending between the first end and the second end. The annular sidewall of the collection container defines an interior reservoir configured to receive a biological sample. The assembly also includes an accessory device removably mounted to the open first end of the collection container. The accessory device includes a cover and a collector. The cover includes an inner portion inserted into the collection container having a sidewall with an inner surface defining a channel through the accessory device and an outer surface with an outer diameter sized to seal against an inner surface of the annular sidewall of the collection container. The cover also includes an outer portion extending radially outward from the inner portion and over the open first end of the collection container. The collector extends distally from the cover and is configured for directing the biological sample through the channel defined by the inner surface of the inner portion of the cover and into the interior reservoir of the collection container.

According to another aspect of the present disclosure, a method for automated blood sample processing includes mounting an accessory device to a collection container by inserting an inner portion of the accessory device though an open first end of the collection container. The accessory device includes a cover and a collector. The cover includes the inner portion, which includes a sidewall having an inner surface defining a channel through the accessory device and an outer surface with an outer diameter sized to seal against an inner surface of a sidewall of the collection container. The cover also includes an outer portion extending radially outward from the inner portion configured to extend over the open first end of the collection container. The collector extends distally from the cover and is configured for directing the blood sample to the channel defined by the inner surface of the inner portion of the cover. The method also includes obtaining the blood sample by directing the blood sample through the collector and into an interior reservoir of the collection container through the channel defined by the inner portion of the accessory device. The method also includes, once the blood sample is obtained, removing the accessory device from the open first end of the collection container and processing the obtained blood sample in the collection container using at least one automated processing device.

Non-limiting examples of the present invention will now be described in the following numbered clauses:
Clause 1: A sample collection accessory device configured for attachment to an open end of a collection container, the accessory device comprising: a cover comprising (i) an inner portion configured for insertion into the collection container comprising a sidewall having an inner surface defining a channel through the accessory device and an outer surface with an outer diameter sized to seal against an inner surface of a sidewall of the container and (ii) an outer portion extending radially outward from the inner portion configured to extend over an end of the sidewall of the collection container; and a collector extending distally from the cover configured for directing a biological sample to the channel defined by the inner surface of the inner portion of the cover.
Clause 2: The accessory device of clause 1, wherein the cover and/or collector comprise a flexible plastic material comprising one or more of silicone, polypropylene, low-density polyethylene, synthetic rubber, or natural rubber.
Clause 3: The accessory device of clause 2, wherein the flexible plastic material comprises additives for increasing wettability of surfaces of the accessory device.
Clause 4: The accessory device of any of clauses 1-3, wherein the inner portion of the cover is tapered, with an outer diameter of a first end of the inner portion being wider than an outer diameter of a second end of the inner portion, and wherein the second end of the inner portion is configured to be inserted into the collection container.
Clause 5: The accessory device of any of clauses 1-4, wherein the channel defined by the inner surface of the inner portion of the cover is tapered with a diameter of a first end of the channel being wider than a diameter of a bottom end of the channel.
Clause 6: The accessory device of any of clauses 1-5, wherein the outer portion comprises a first end connected to a first end of the inner portion, a second end, and an annular sidewall extending between the first end and the second end of the outer portion.
Clause 7: The accessory device of clause 6, wherein an outer surface of the inner portion and an inner surface of the sidewall of the outer portion define an annular slot sized to receive a lip feature extending from the open end of the collection container.
Clause 8: The accessory device of any of clauses 1-7, wherein an axial length of the inner portion is longer than an axial length of the outer portion.
Clause 9: The accessory device of any of clauses 1-8, wherein the accessory device and collection container are configured for collection and storage of a capillary blood sample.
Clause 10: The accessory device of any of clauses 1-9, wherein the collector comprises at least one of a scoop, a funnel, or a capillary action member.
Clause 11: The accessory device of any of clauses 1-10, wherein the collector comprises at least one of a rod, a hollow tube, or a plastic film configured to draw the sample through the accessory device by capillary action.
Clause 12: The accessory device of any of clauses 1-11, wherein the cover and the collector are an integral part formed by a single molding process, such as injection molding.
Clause 13: The accessory device of any of clauses 1-12, wherein the collector comprises a scoop comprising (i) a first portion connected to and extending from the inner portion of the cover for guiding the sample toward the inner portion of the cover, (ii) a second portion opposite the first portion, and (iii) a guiding surface extending between the first portion and the second portion.
Clause 14: The accessory device of clause 13, wherein the second portion of the scoop comprises a curved top edge, which curves in at least two dimensions.
Clause 15: The accessory device of any of clauses 1-10, wherein the collector comprises a capillary action rod mounted to the cover by a wall extending radially between an outer surface of the capillary action rod and the inner surface of the inner portion of the cover.
Clause 16: The accessory device of clause 15, wherein the rod extends through the channel defined by the inner surface of the inner portion of the cover, and wherein the rod is hollow comprising an open first end, an open second end, and an annular sidewall extending between the first end and the second end.
Clause 17: The accessory device of clause 16, wherein the sidewall of the rod defines a narrow channel extending from the first end to the second end of the rod sized for capillary action of the biological sample through the narrow channel.
Clause 18: The accessory device of any of clauses 15-17, wherein the capillary action rod extends axially beyond a first end of the cover in a distal direction and extends axially beyond a second end of the cover in a proximal direction.
Clause 19: The accessory device of any of clauses 1-10, wherein the collector comprises a tubular body defining a channel configured to guide the sample to the channel of the cover and a connector on an exterior portion of the collector configured to engage a corresponding connector of a puncture device for removably connecting the puncture device to the accessory device.
Clause 20: The accessory device of clause 19, wherein, when engaged to the connector on the exterior portion of the collector, the puncture device is positioned over the channel defined by the tubular body for directing the biological sample through the tubular body and cover into an interior reservoir of the collection container.
Clause 21: The accessory device of clause 20, wherein the biological sample moves from the puncture device through the tubular body of the connector and cover into the interior reservoir of the collection container by gravity.
Clause 22: The accessory device of any of clauses 19-21, wherein the connector is configured to rotatably engage the puncture device, such that the puncture device is movable from a pre-use position, where the puncture device is spaced apart from the open end of the collection container, to an in-use position, wherein the puncture device covers the open end of the collection container.
Clause 23: The accessory device of any of clauses 19-22, wherein the connector comprises a cylindrical member configured for insertion into a corresponding receptor of the puncture device.
Clause 24: The accessory device of clause 23, wherein the cylindrical member is retained within the receptor of the sample collection container by a snap fit connection.
Clause 25: The accessory device of clause 24, wherein the collector further comprises a latch configured to engage a portion of the puncture device for maintaining the puncture device in an in-use position with the puncture device covering the open end of the collection container.
Clause 26: A collection assembly comprising: a collection container comprising an open first end, a second end, and an annular sidewall extending between the first end and the second end, the annular sidewall of the collection container defining an interior reservoir configured to receive a biological sample; and an accessory device removably mounted to the open first end of the collection container, the accessory device comprising: a cover comprising (i) an inner portion inserted into the collection container comprising a sidewall having an inner surface defining a channel through the accessory device and an outer surface with an outer diameter sized to seal against an inner surface of the annular sidewall of the collection container and (ii) an outer portion extending radially outward from the inner portion and over the open first end of the collection container; and a collector extending distally from the cover configured for directing the biological sample through the channel defined by the inner surface of the inner portion of the cover and into the interior reservoir of the collection container.
Clause 27: The collection assembly of clause 26, wherein the collector of the accessory device comprises at least one of a scoop, a funnel, or a capillary action member.
Clause 28: The collection assembly of clause 26 or clause 27, wherein the collector of the accessory device comprises a scoop comprising (i) a first portion connected to and extending from the inner portion of the cover for guiding the sample toward the inner portion of the cover, (ii) a second portion, and (iii) a guiding surface extending between the first portion and the second portion.
Clause 29: The collection assembly of clause 26 or clause 27, wherein the collector of the accessory device comprises a capillary action rod mounted to the cover by a wall extending radially between an outer surface of the capillary action rod and the inner surface of the inner portion of the cover.
Clause 30: The collection assembly of any of clauses 26-29, wherein the collection container is a micro-tube with the interior reservoir having a volume of about 600 µL to 1000 µL.
Clause 31: The collection assembly of clause 26, further comprising a blood collection puncture device rotatably mounted to the accessory device configured to rotate about a connector of the accessory device between a pre-use position in which the puncture device is spaced apart from the open first end of the collection container and an in-use position where the puncture device is over the open first end of the collection container.
Clause 32: The collection assembly of clause 31, wherein the biological sample moves from the puncture device through the collector and cover of the accessory device into the interior reservoir of the collection container by gravity.
Clause 33: The collection assembly of clause 31 or clause 32, wherein the connector comprises a cylindrical member that is inserted into a corresponding receptor of the puncture device.
Clause 34: The collection assembly of clause 33, wherein the cylindrical member is retained within the receptor of the sample collection container by a snap fit connection.
Clause 35: The collection assembly of clause 33 or clause 34, wherein the collector further comprises a latch that engages a portion of the puncture device for maintaining the puncture device in the in-use position with the puncture device covering the open first end of the collection container.
Clause 36: The collection assembly of any of clauses 31-35, wherein the puncture device comprises a receptor sized to receive the connector of the accessory device for securing the puncture device to the cap.
Clause 37: The collection assembly of any of clauses 31-36, wherein the receptor comprises an annular member defining an opening sized to receive the connector of the accessory device.
Clause 38: The collection assembly of any of clauses 31-37, wherein the puncture device comprises: a holder for receiving a sample source, the holder having an actuation portion and a port; a lancet housing secured within the port, the lancet housing having an inlet and an interior; and a puncturing element moveable between a pre-actuated position, in which the puncturing element is retained within the interior, and a puncturing position, in which at least a portion of the puncturing element extends through the inlet.
Clause 39: A method for automated blood sample processing, comprising: mounting an accessory device to a collection container by inserting an inner portion of the accessory device though an open first end of the collection container, wherein the accessory device comprises: a cover comprising (i) the inner portion comprising a sidewall having an inner surface defining a channel through the accessory device and an outer surface with an outer diameter sized to seal against an inner surface of a sidewall of the collection container and (ii) an outer portion extending radially outward from the inner portion configured to extend over the open first end of the collection container; and a collector extending distally from the cover configured for directing the blood sample to the channel defined by the inner surface of the inner portion of the cover; obtaining the blood sample by directing the blood sample through the collector and into an interior reservoir of the collection container through the channel defined by the inner portion of the accessory device; once the blood sample is obtained, removing the accessory device from the open first end of the collection container; and processing the obtained blood sample in the collection container using at least one automated processing device.
Clause 40: The method of clause 39, further comprising, after removing the accessory device from the collection container and prior to processing the blood sample, attaching a cap over the open first end of the collection container to seal the blood sample within the interior reservoir of the collection container.
Clause 41: The method of clause 39 or clause 40, wherein the collector of the accessory device comprises at least one of a scoop, a funnel, or a capillary action member.
Clause 42: The method of any of clauses 39-41, wherein the collector of the accessory device comprises a scoop comprising (i) a first portion connected to and extending from the inner portion of the cover for guiding the blood sample toward the inner portion of the cover, (ii) a second portion, and (iii) a guiding surface extending between the first portion and the second portion.
Clause 43: The method of any of clauses 39-41, wherein the collector of the accessory device comprises at least one of a rod, a hollow tube, or a plastic film configured to draw the blood sample through the accessory device by capillary action.
Clause 44: The method of any of clauses 39-43, wherein processing the blood sample in the collection container comprises one or more of the following automated testing processes: sorting collection containers containing blood samples by type, size, and/or contents with an automated sorting machine; filling selected specimen collection containers with specific additives; centrifugation; vision-based specimen quality analysis; serum level analysis; decapping; aliquoting; and/or automated labeling of collection containers.
Clause 45: The method of any of clauses 39-44, wherein obtaining the blood sample comprises obtaining a capillary blood sample by pricking a skin surface of a patient and drawing the blood sample into the interior reservoir of the collection container with the collector of the accessory device.
Clause 46: The method of any of clauses 39-41, wherein the collector of the accessory device comprises a tubular body defining a channel configured to guide the blood sample to the channel of the cover and a connector on an exterior portion of the collector configured to engage a corresponding connector of a puncture device for removably securing the puncture device to the accessory device.
Clause 47: The method of clause 46, further comprising, after mounting the accessory device to the collection container, attaching the puncture device to the collector by inserting the connector of the collector through a corresponding receptor of the puncture device.
Clause 48: The method of clause 47, wherein the puncture device comprises: a holder for receiving a sample source, the holder having an actuation portion and a port; a lancet housing secured within the port, the lancet housing having an inlet and an interior; and a puncturing element moveable between a pre-actuated position, in which the puncturing element is retained within the interior, and a puncturing position, in which at least a portion of the puncturing element extends through the inlet.
Clause 49: The method of clause 47 or clause 48, wherein the connector is rotatably engaged to the receptor via a snap fit connection.
Clause 50: The method of any of clauses 47-49, further comprising moving the puncture device from a pre-use position, in which the puncture device is spaced apart from the open first end of the collection container, to an in-use position, in which the puncture device covers the open first end of the collection container.
Clause 51: The method of any of clauses 39-50, wherein the collection container comprises the open first end, a second end, and an annular sidewall extending between the first end and the second end, the annular sidewall of the collection container defining the interior reservoir configured to receive the blood sample.
Clause 52: The method of any of clauses 39-51, wherein the collection container comprises a micro-tube with the interior reservoir having a volume of about 600 µL to 1000 µL.

### BRIEF DESCRIPTION OF THE DRAWINGS

FIG. 1A is a perspective view of a sample collection assembly including a sample collection container and accessory mounted to the container, according to an aspect of the present disclosure.
FIG. 1B is a perspective view of a cross-section of a top portion of the sample collection assembly of FIG. 1A.
FIG. 1C is an exploded view of a cross-section of portions of the sample collection assembly of FIG. 1A.
FIG. 2A is a perspective view of another example of a sample collection assembly including a sample collection container and accessory mounted to the container, according to an aspect of the present disclosure.
FIG. 2B is a perspective view of a cross-section of a top portion of the sample collection assembly of FIG. 2A.
FIG. 2C is an exploded view of a cross-section of portions of the sample collection assembly of FIG. 2A.
FIG. 3A is a perspective view of another example of a sample collection assembly including a sample collection container and accessory mounted to the container, according to an aspect of the present disclosure.
FIG. 3B is a perspective view of a cross-section of a portion of the collection container and accessory of FIG. 3A.
FIG. 3C is an exploded view of a cross-section view of portions of the sample collection assembly of FIG. 3A.
FIG. 4A is a perspective view of the sample collection assembly of FIG. 3A and a puncture device prior to connecting the puncture device to the accessory of the sample collection assembly, according to an aspect of the present disclosure.
FIG. 4B is an exploded perspective view of the collection assembly and puncture device of FIG. 4A, according to an aspect of the present disclosure.
FIG. 4C is a perspective view of the collection assembly and puncture device of FIG. 4A, with the puncture device mounted to the accessory in an open or pre-use position, according to an aspect of the present disclosure.
FIG. 4D is a perspective view of the collection assembly and puncture device of FIG. 4A, with the puncture device mounted to the accessory in a closed or in-use position, according to an aspect of the present disclosure.
FIG. 5A is an exploded perspective view of a small volume sample collection container assembly for collecting biological samples which can be used with an accessory, according to an aspect of the present disclosure.
FIG. 5B is a cross-sectional view of the sample collection assembly of FIG. 5A in a pre-centrifugation condition.
FIG. 5C is a cross-sectional view of the sample collection assembly of FIG. 5A in a post-centrifugation condition.
FIG. 5D is another cross-sectional view of the capillary collection assembly of FIG. 5A showing various dimensions of the assembly.
FIG. 6A is a perspective view of a cap which can be attached to an open top end of the sample collection container of FIG. 5A, according to an aspect of the present disclosure.
FIG. 6B is a cross-sectional view of the cap of FIG. 6A.
FIG. 7A is a perspective view of a sample collection assembly including a puncture device for obtaining a blood sample from a patient's finger, according to an aspect of the present disclosure.
FIG. 7B is a cross-sectional view of the puncture device of FIG. 7A including a lancet, according to an aspect of the present disclosure.
FIG. 7C is a perspective view of a holder of the puncture device of FIG. 7A, according to an aspect of the present disclosure.
FIG. 7D is a schematic drawing showing a top view of the holder of FIG. 7C connected to a patient's finger for performing a blood collection procedure.
FIG. 7E is another schematic drawing showing a front view of the holder of FIG. 7C connected to the patient's finger.
FIG. 8 is a flow chart showing a method for sample collection and automated processing using a small volume sample collection container and accessory, according to an aspect of the present disclosure.

### DETAILED DESCRIPTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. The term "proximal" refers to a central or interior portion of a device or object. For example, a "proximal" end of an elongated member may refer to the end of the member that is connected to a central portion or body of a device. By contrast, the term "distal" refers to an outer portion of a device or object that is spaced apart from a central portion or body of the device or object. For example, a "distal" end of an elongated member may refer to the end of the member that is farthest from the body of the device and is not connected to other portions of the device. However, it is to be understood that the invention may assume alternative variations and step sequences, except where expressly specified to the contrary. It is also to be understood that the specific devices and processes illustrated in the attached drawings, and described in the following specification, are simply exemplary embodiments of the invention. Hence, specific dimensions and other physical characteristics related to the embodiments disclosed herein are not to be considered as limiting.

The present disclosure relates to biological sample collection assemblies 10 and systems for collecting biological samples, such as capillary blood samples and other fluid samples, including a sample collection container 12 or collection tube for receiving the biological sample. Biological samples can be collected from a patient by a medical professional or practitioner trained for obtaining biological samples, such as blood samples. The practitioner can be a clinician or healthcare worker that performs blood draw, fluid delivery, and/or infusion procedures for patients. For example, the "healthcare worker" can be a medical technician, phlebotomist, nurse, or another practitioner trained to perform the blood draw procedure in accordance with sterile practices and protocols of a medical facility. The collection assemblies 10 disclosed herein can also include an accessory 28 or accessory device configured to be connected to the sample collection container 12 for introducing or guiding the biological sample into an interior of the container 12. The assemblies 10, containers 12, and accessories 28 disclosed herein can be configured to allow for improved blood collection from a puncture site into an interior of the container 12, which can be especially important for sample collection from difficult to access capillary stick sites, such as for infant heel-sticks.

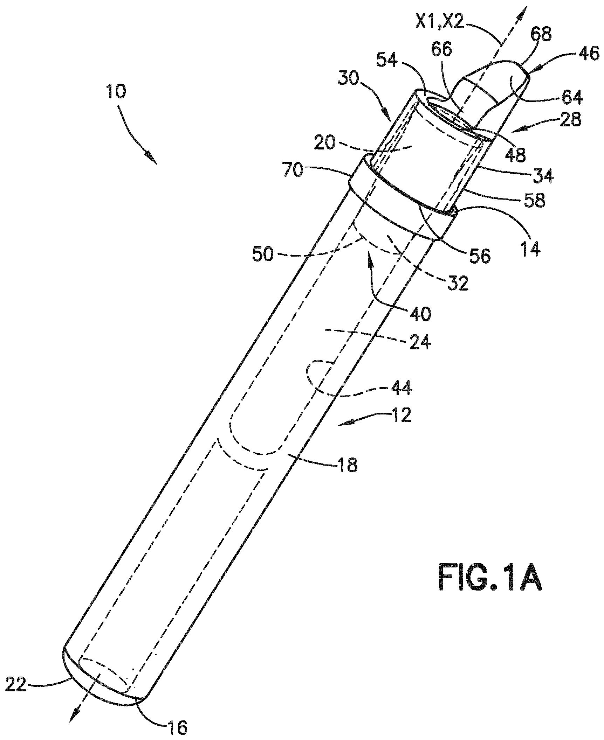

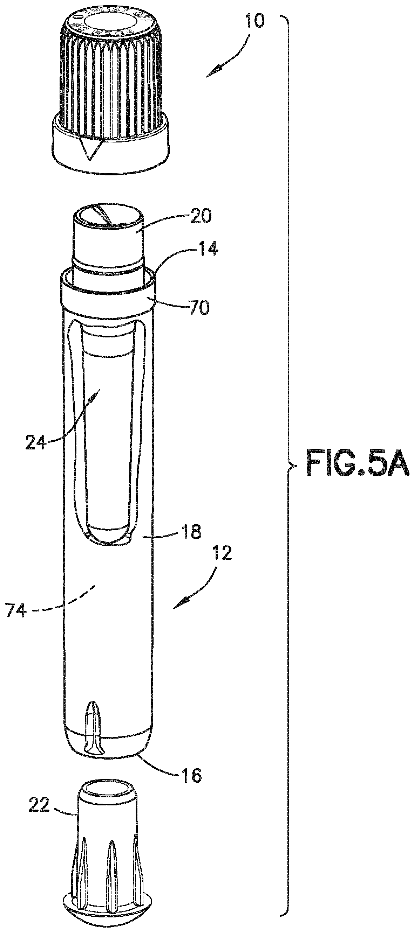

The accessories 28 of the present disclosure can be used with many different types of sample collection assemblies 10, containers 12, and micro-tubes known in the art. Examples of sample collection assemblies that can be used with the accessories 28 are described, for example, in U. S. Patent Appl. Pub. No. 2022/0280080 entitled "Small Volume Collection Container", which is incorporated by reference herein in its entirety. In some examples, as shown, for example, in FIG. 1A, the sample collection container 12 comprises a body or tube having a first or top open end 14, a second or bottom end 16, which can be closed or open, and an annular sidewall 18 extending therebetween. The open top end 14 can be a suitable size and/or shape for collecting samples from a puncture location, such as a skin surface of a patient. As described in further detail herein, in some examples, the open top end 14 can have a flat, 360° lip feature 20 (shown in FIGS. 5A-5D) surrounding the open top end 14 of the container 12. When the bottom end 16 of the container 12 is open, the sample collection container 12 can also include a plug 22 configured to be coupled to the bottom end 14 of the collection container 12 to close the bottom end 14 of the collection container 12. The annular sidewall 18 and/or any interior walls of the collection container 12 can define an interior or interior reservoir 24 of the container 12 sized to contain a volume of a biological sample sufficient for a diagnostic test to be performed. For example, the collection container 12 can be a micro-tube with the internal reservoir 24 having a volume of about 600 µL to 1000 µL. The collection assembly 10 can also include a cap 26 (shown in FIGS. 5A-6B) configured to be connected to the collection container 12 to close the open top end 14 of the collection container 12 for securing the biological sample in the interior reservoir 24 of the container 12.

The sample collection containers 12 are configured for receipt and storage of a biological sample for analysis and testing. As used herein, a "biological sample" can refer to a sample comprising one or more biological fluids (e.g., liquid(s), mixture(s), and/or solution(s) obtained from a patient) including, but not limited to, blood (e.g., arterial blood, venous blood, capillary blood, etc.), plasma, saliva, urine, or other fluids obtained from a patient. As used herein, the term "patient" can mean a mammalian organism, and the collection container 10 of the present disclosure can be intended for use in specimen collection procedures performed on humans and/or animals. The collection container 12 can be used for collection, storage, and eventual transfer of the biological sample for purposes of diagnostic testing. For example, the collection container 12 can be configured to be used for proteomics, molecular diagnostics, chemistry sampling, blood, or other bodily fluid collection, coagulation sampling, hematology sampling, and the like.

In some examples, the collection containers 12 and accessories 28 disclosed herein are configured to improve biological sample collection for automation compatible small volume sample collection containers, where a geometry of the container is already challenged by the design constraints for compatibility with multiple front-end automation and analyzer systems. These constraints can cause collection containers 12 to have geometries that are not suitable for conventional capillary blood collection and, therefore, dictate a need for the accessories 28 to improve sample collection. For these reasons, an objective of the accessories 28 is to improve ease of biological sample collection for automation compatible small volume collection containers 12, including improved blood flow, elimination of red cell hang up in the open end of the collection container 12, hemolysis, and other sample quality related factors that can typically be an issue in capillary blood.

More specifically, the collection container 12 having the flat open top end 14 or lip feature 20 can be generally symmetrical about a longitudinal axis X1 thereof, meaning that the collection container 12 can be inserted into an automated processing machine in many or all orientations. The collection containers 12 with the flat open top end 14 can be used with automated front end processes used to prepare a specimen for proper analysis, such as automated sorting machines and other types of automated devices. These devices can include, for example, devices for filling selected specimen collection containers with specific additives, centrifugation, vision-based specimen quality analysis, serum level analysis, decapping, aliquoting, and/or automated labeling of containers. The collection containers 12 can also be compatible with automated analyzing procedures and to accommodate automated diagnostic and/or analyzing probes or other specimen extraction equipment. The collection containers can also be compatible with automated back end processes employed after a specimen is analyzed, such as resealing, storage, and retrieval.

In some examples, the collection containers of the present disclosure are usable with different commercially available automated sample analyzers. Examples of such analyzers can include, but are not limited to, the cobas^{®} P512, cobas^{®} P612, cobas^{®} c501, cobas^{®} c502, cobas^{®} c602, cobas^{®} e601, cobas^{®} e602, cobas^{®} e801, and/or cobas^{®} e802 from Roche Diagnostics, the DxA, AU5800, DCX700AU, Dxl, and/or Falcon from Beckman Coulter, Inc., the Atellica^{®} SH, Aptio^{®}, Atellica^{®} CH, and/or Atellica^{®} IA from Siemens Healthcare, the Vitros^{®} from Ortho Clinical Diagnostics, and/or the GLP, a3600, Alinity, and/or Architect from Abbott.

The present disclosure is also related to the accessories 28 or accessory devices for use with the collection containers 12 that facilitate or simplify obtaining, collecting, storing, and/or manipulating collected samples. More specifically, the accessories 28 of the present disclosure can include various caps, closures, covers, lids, and other sealing members configured to be positioned over the open top end 14 of the collection container 12. The accessories 28 can include one or more structures that direct the biological sample from a collection location, such as a puncture site on a patient's skin, to the interior reservoir 24 of the collection container 12. In some examples, the accessories 28 can be single-use disposable devices configured to be attached to an open top end 14 of the collection container 12 before collection and discarded after collection.

In some examples, the accessories 28 can include a plastic collection device including a cap portion that connects to the open top end 14 of the collection container 12. The accessories 28 can also include a distally-extending collector, such as an extension apparatus, extending from the cap portion configured to cause the biological sample to flow from a sample collection site (e.g., a skin prick or vascular access site) into the interior reservoir 24 of the collection container 12. The accessories 28 can also include other mechanical flow inducing structures, which can be connected to the open top end 14 of the collection container 12, such as glass or plastic capillary-action structures (e.g., such as tubes, rods) or plastic films, attached to the open top end 14 or interior of the collection container 12 for drawing the biological sample towards the interior reservoir 24 of the collection container 14.

### Accessory for Blood Sample Collection Including a Scoop

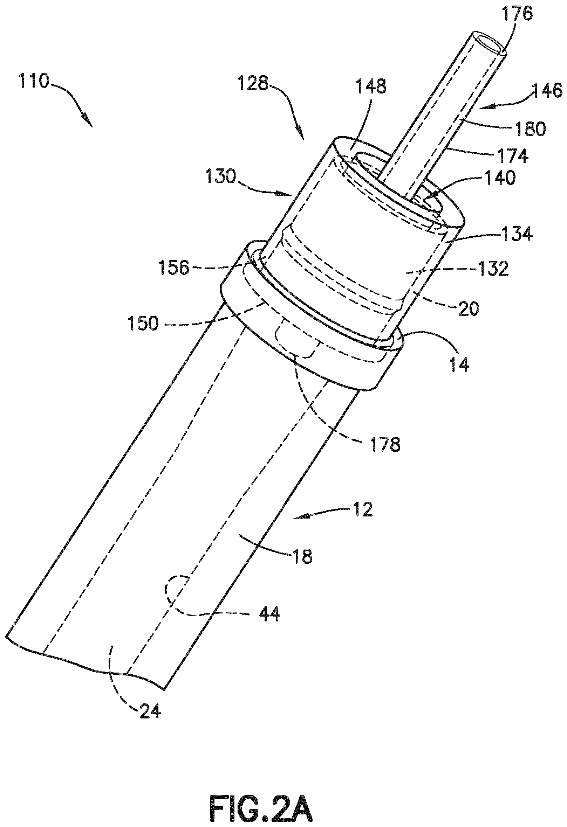

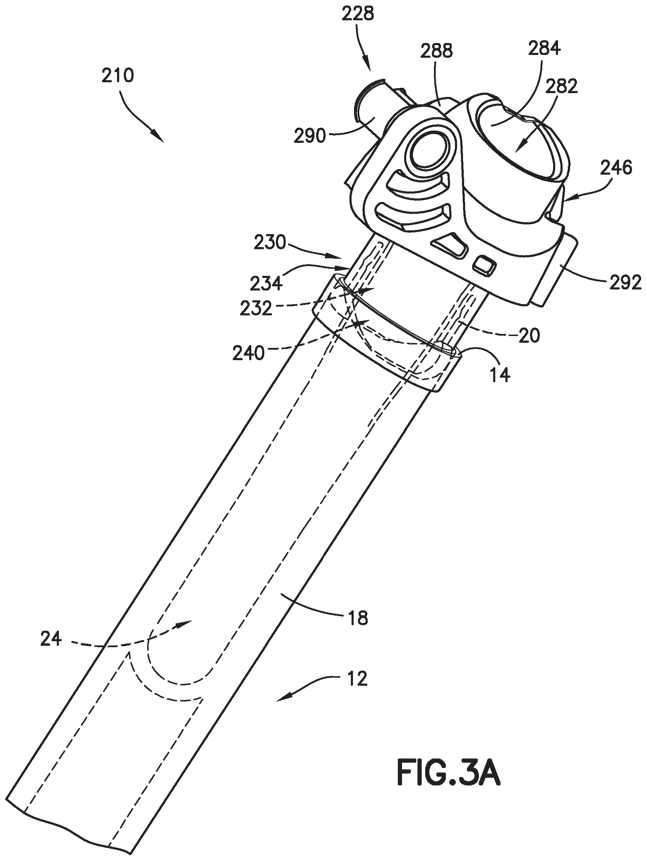

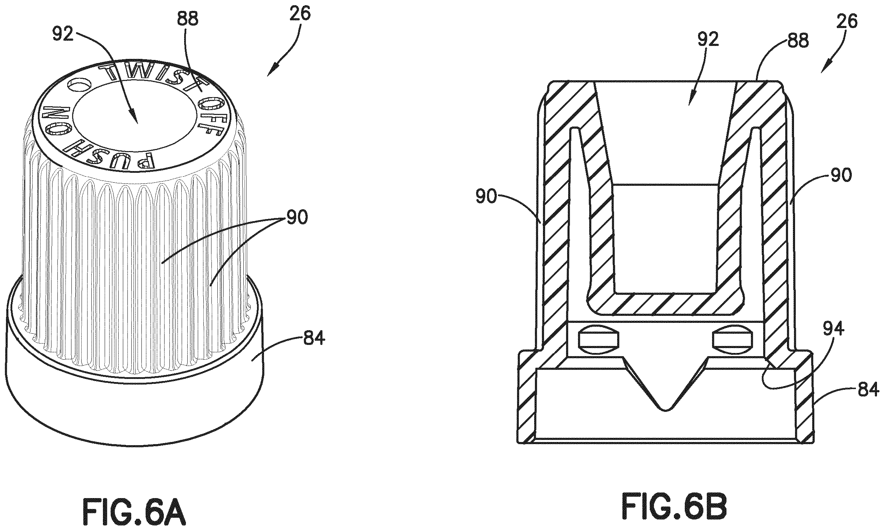

The biological sample collector assemblies 10 disclosed herein include the removable sample collection accessory 28 adapted to facilitate sample collection, examples of which are shown in FIGS. 2A-2C. Additional accessories 128, 228 are shown in FIGS. 3A-5B. The accessories 28, 128, 228 are configured to be removably mounted or attached to the open top end 14 of the collection container 12 during collection of the biological sample. For example, the accessories 28, 128, 228 can include guiding surfaces or members configured to direct the biological sample from a patient (e.g., from a puncture location) through the accessory 28, 128, 228 and into the interior of the collection container 12. The biological sample can move through the accessories 28, 128, 228 by gravity, capillary forces, or by other passive or active transport forces for movement of a small volume fluid sample from a first location to a second location. Once the biological sample is obtained and moved to the interior of the collection container, the accessories 28, 128, 228 can be removed and the collection container 12 can be capped (e.g., with a cap 26 shown, for example, in FIGS. 6A-7C) until the biological sample is ready to be tested.

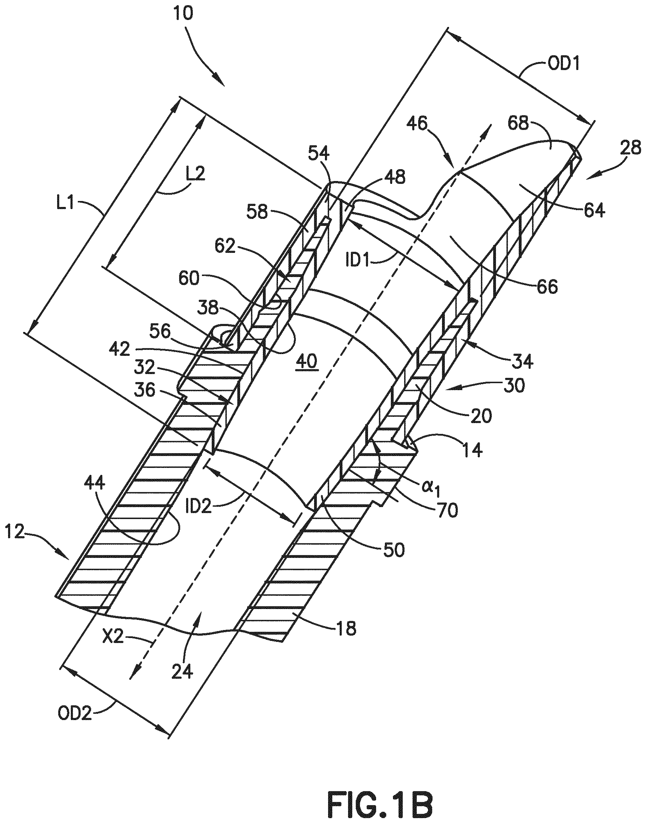

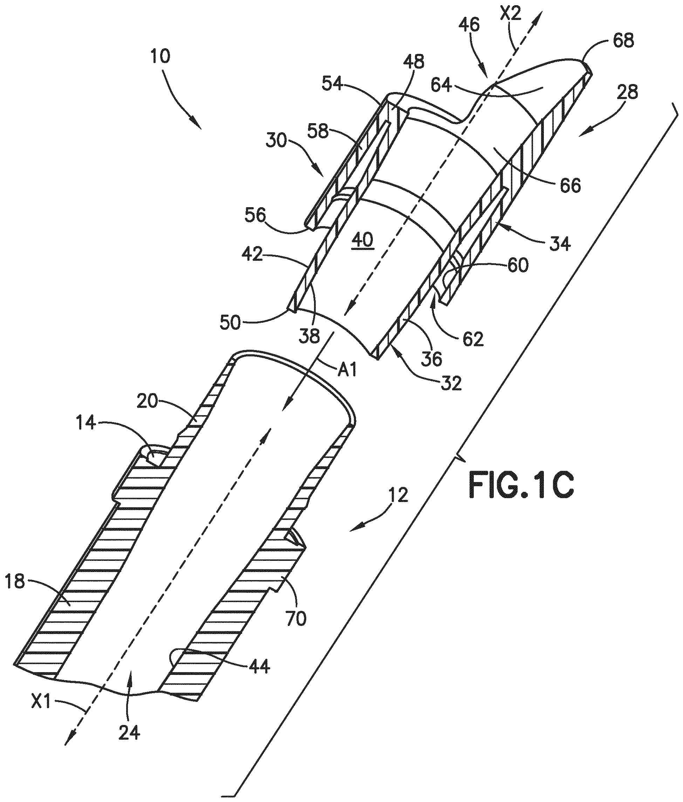

As shown in FIGS. 1A-1C, the sample collection accessory 28 of the sample collection assembly 10, which includes geometries that facilitate collection of the biological sample (e.g., a capillary blood sample), is configured to be attached to the open top end 14 of the collection container 12. The accessory 28 comprises a cap, cap portion, or cover 30 configured to be positioned over the open top end 14 of the collection container 12. The cover 30 can have a concentric arrangement with an inner annular member or inner portion 32 configured for insertion into the collection container 12 and an outer annular member or outer portion 34 enclosing the inner portion 32. The inner portion 32 can include a sidewall 36 having an inner surface 38 defining a fluid or flow channel 40, such as fluid channel suitable for permitting the collected biological sample to pass through the channel 40 and into the interior reservoir 24 of the collection container 12 through the accessory 28, and an outer surface 42 with a maximum or widest outer diameter OD1 (shown in FIGS. 1B and 1C) sized to contact, press against, and/or seal an inner surface 44 of the sidewall 18 of the collection container 12. As shown in FIGS. 1A-1C, the outer portion 34 of the cover 30 extends over and/or about the open top end 14 of the collection container 12 preventing fluid from passing into or from the interior reservoir 24 of the collection container 12 except through the channel 40 defined by the inner portion 32 of the cover 30. The accessory 28 also includes a collector 46 extending distally from the cover 30 configured for directing the biological sample to the channel 40 defined by the inner surface 38 of the inner portion 32.

The cover 32 is generally shaped to direct flow of the biological sample into the interior reservoir 24 of the collection container 12. For example, as shown in FIGS. 1A-1C, the inner portion 32 of the cover 30 can be tapered (e.g., can have a tapered inner surface 38 and/or outer surface 42). For example, the maximum outer diameter OD1 of a first or top end 48 of the inner portion 32 can be wider than an outer diameter OD2 (shown in FIGS. 1B and 1C) of a second or bottom end 50 of the inner portion 32, which is sized to be inserted into the collection container 12. Having a tapered outer surface 42 of the inner portion 32 can make it easier to slide the accessory 28 through the open top end 14 of the collection container 12. The channel 40 defined by the inner surface 38 of the inner portion 32 of the cover 30 can also be tapered with an inner diameter ID1 at the top end 48 of the inner portion 32 being wider than an inner diameter ID2 at the bottom end 50 of the inner portion 32. The amount of taper and/or dimensions of the top end 48 and the bottom end 50 of the inner portion 32 can be selected to ensure that fluid flows efficiently through the channel 40. In particular, the taper angle can be steep enough to ensure that the biological sample moves through the channel 40 in a controlled manner. However, the bottom end 50 of the inner portion 32 desirably is wide enough to ensure that solid components of a collected biological sample, such as platelets, minerals, and other particles, do not adhere to the inner surface 38 of the inner portion 32 or otherwise block fluid flow through the accessory 28.

The outer portion 34 of the cover 30 can be an annular or tubular structure having a first or top end 54, an open second or bottom end 56 opposite the top end 54, and a sidewall 58 extending about the inner portion 32 between the top end 54 and the bottom end 56 of the outer portion 34. The top end 54 of the outer portion 34 can be connected to or integral with the top end 48 of the inner portion 32. Further, as shown in FIGS. 1B and 1C, the outer surface 42 of the inner portion 32 and an inner surface 60 of the outer portion 34 define an annular slot 62 sized to receive the open top end 14 of the collection container 12. In some examples, contact between the inner surface 60 of the outer portion 34 and/or the outer surface 42 of the inner portion 32 and the collection container 12 can be sufficient for holding the accessory 28 in place preventing the accessory 28 from sliding out of the collection container 12 at an unexpected or inconvenient time. As previously described, dimensions of the accessory 28 are generally dependent upon dimensions of the collection container 12 and/or automated processing machines that are used with the collection container.

The accessory 28 also includes the collector 46 extending distally from the cover 30 configured for directing the biological sample being collected to the channel 40 defined by the inner surface 38 of the inner portion 32 of the cover 30. The collector 46 can be any structure configured for directing the biological sample from, for example, a puncture location (e.g., skin prick location) on the patent's skin towards the channel 40 of the cover 30. For example, the collector 46 can include angled, arcuate, curved, or tapered surfaces for directing the biological sample toward the channel 40. In other examples, the collector 46 can include tubular and/or elongated members for directing the fluid sample towards the channel 40 and/or interior reservoir 24 of the collection container 12. In particular, in some examples, the collector 46 can comprise one or more of a scoop, wedge, funnel, and/or a capillary-action device (e.g., a rod, tube, film, or similar structure) for directing the biological sample toward the channel 40 and/or towards the interior reservoir 24 of the collection container 12.

The cover 30 and/or collector 46 can be formed from or comprise a flexible plastic and/or elastomeric material, such as silicone, polypropylene, low-density polyethylene, synthetic rubber (e.g., polychloroprene), natural rubber (e.g., isoprene), as well as from a harder and/or more rigid material, such a rigid thermoplastic material, such as acrylonitrile butadiene styrene (ABS), polyester, polycarbonate, polypropylene, high-density polyethylene, or polyethylene terephthalate, as well as from glass and other ceramic materials. In some examples, the accessory 28 can be a single molded part made by a conventional molding process, such as injection molding. In such instances, the inner portion 32 and the outer portion 34 of the cover 30 can be integral with the collector 56. For example, a guiding surface 64 of the collector 56 can seamlessly extend distally from the inner surface 38 of the inner portion 32 of the cover 30 without gaps, joints, edges, or any other structures that would disrupt flow of the biological sample through the accessory 28 to the interior reservoir 24 of the collection container 12. In some examples, the inner surface 38 of the inner portion 32 and/or other surfaces of the accessory 28 can include additive materials for increasing wettability of the surfaces of the accessory 28. The additive materials can be added to the plastic or precursor material for making the accessory 28 prior to molding and/or can be applied to surfaces of a molded part after molding and/or curing.

With continued reference to FIGS. 1A-1C, in some examples, the collector 46 of the accessory 28 is a scoop configured to press against the patient's skin providing a flow path from a puncture location of the skin, through the accessory 28, and to the interior reservoir 24 of the collection container 12. By using the scoop, the practitioner is able to effectively acquire a blood sample allowing a sufficient amount of the sample to pass through the accessory 28 to the collection container 14. In some examples, the collector 46 or scoop can include a first, proximal, or bottom portion 66 connected to and extending from the inner portion 32 for guiding the sample toward the channel 40 extending through the inner portion 32 of the cover 30. The collector 46 or scoop also includes a second, distal, or top portion 68 opposite the bottom portion 66. The guiding surface 64 is an inwardly facing surface extending between the bottom portion 66 and the top portion 68. The top portion 68 of the collector 46 or scoop can further comprise a curved top edge, which is curved radially about a longitudinal axis X2 of the accessory 28 and about an axis substantially transverse to the longitudinal axis X2 of the accessory 28. The guiding surface 64 is provided for directing the biological sample towards the channel 40 defined by the inner surface 38 of the inner portion 32. The guiding surface 64 can be a curved surface (e.g., a surface that curves in multiple directions) sloping from the top portion 68 or top edge of the collector 46 towards the longitudinal axis of the container 12 and accessory 28. In other examples, the guiding surface 64 can be a flat or planar angled surface that does not slope or curve towards a central axis or portion of the guiding surface 64.

As previously described, the accessory 28 is removably inserted into the open top end 14 of the collection container 12. The accessory 28 is shown detached from the collection container 12 in FIG. 1C. In order to mount the accessory 28 to the open top end of the collection container 12, the practitioner pushes the bottom end 50 of the inner portion 32 of the accessory 28 through the open top end 14 of the collection container 12 as shown by arrow A1 (in FIG. 1C), such that the outer surface 42 of the inner portion 32 of the cover 30 contacts the inner surface 44 of the sidewall 18 of the collection container 12. The practitioner then advances the inner portion 32 into the interior reservoir 24 of the collection container 12 bringing the bottom end 56 of the outer portion 34 into contact with an annular ridge 70 or ring extending radially outward from the sidewall 18 of the collection container 12. As previously described, the top end 14 of the collection container 12 slides into the slot 62 defined between the outer surface 42 of the inner portion 32 and the inner surface 60 of the outer portion 34. When the accessory 28 is mounted to the collection container 12, the collector 46, such as the scoop, extends distally from the top end 48, 54 of the inner or outer portions 32, 34 of the cover 30, as shown in FIGS. 1A and 1B.

In contrast to the collector assembly 10 including the removable accessory 28 of the present disclosure, many conventional collection containers or tubes have an open top end with one or multiple collector structures, such as scoops, extending distally from the top lip portion of the collection container or tube. These conventional collectors or scoops typically are integral with and extend distally from the top lip portion of the container or tube. However, containers including integral scoops can be complicated to manufacture requiring complex or additional tooling to form the scoops than is needed for a container or tube with a flat open top end. Containers with integral scoops also may not work well with automated processing machines because such containers may need to be positioned with the scoops in a particular orientation in order to be recognized and/or manipulated by the automated machine. For example, vision systems of some automated machines may only recognize the container when the scoops are in a specific orientation. However, needing to ensure that each container is properly positioned prior to using the automated machine increases time and manual effort needed for processing filled collection containers. As such, it is believed that collection assemblies 10 including the removable and/or discardable accessories 28 disclosed herein provide substantial benefits in terms of manufacturability and universal automated processing compared to conventional collection assemblies and containers.

### Accessory for Blood Sample Collection with a Capillary Action Device

FIGS. 2A-2C show another example of a sample collection assembly 110 including an accessory 128 configured to be mounted to the open top end 14 of the collection container 12. As in previous examples, the accessory 128 comprises a cover 130 that includes an inner portion 132 configured for insertion into the interior reservoir 24 of the collection container 12. The inner portion 132 of the cover 130 includes a sidewall 136 with an inner surface 138 defining a channel 140 through the accessory 128 and an outer surface 142 with an outer diameter sized to seal against the inner surface 44 of the sidewall 18 of the collection container 12. The cover 130 also includes the outer portion 134 extending over the open top end 14 of the collection container 12.

The accessory 128 also includes a collector 146 extending distally from the cover 130 configured for directing the biological sample to the channel 140 defined by the inner surface 138 of the inner portion 132 of the cover 130. However, unlike in previous examples, the collector 146 of the accessory 128 in FIGS. 2A-2B is a capillary action device, such as a rod, post, or plastic film, configured for drawing or inducing flow of the biological sample along surface(s) of the capillary action device for transporting the biological sample, such as a capillary blood sample, from a puncture location on the patient's skin into the interior reservoir 24 of the collection container 12.

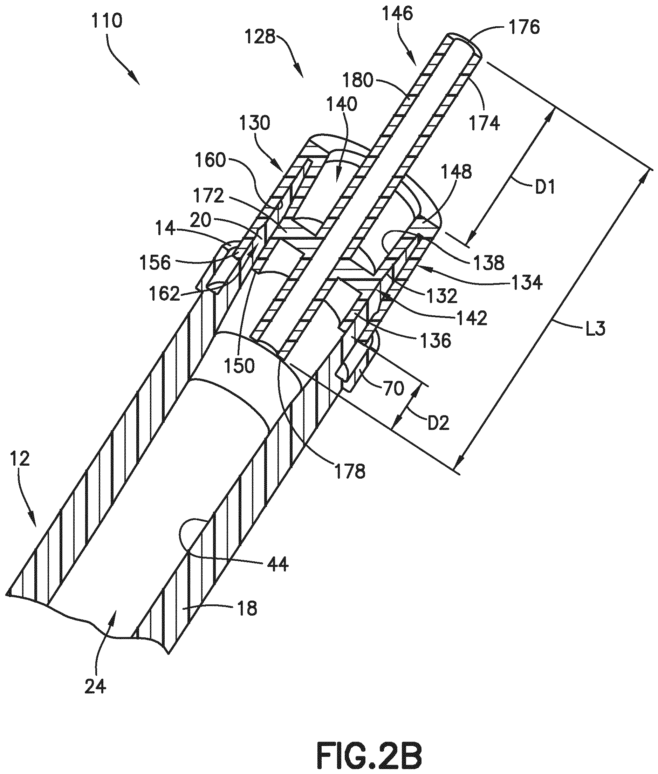

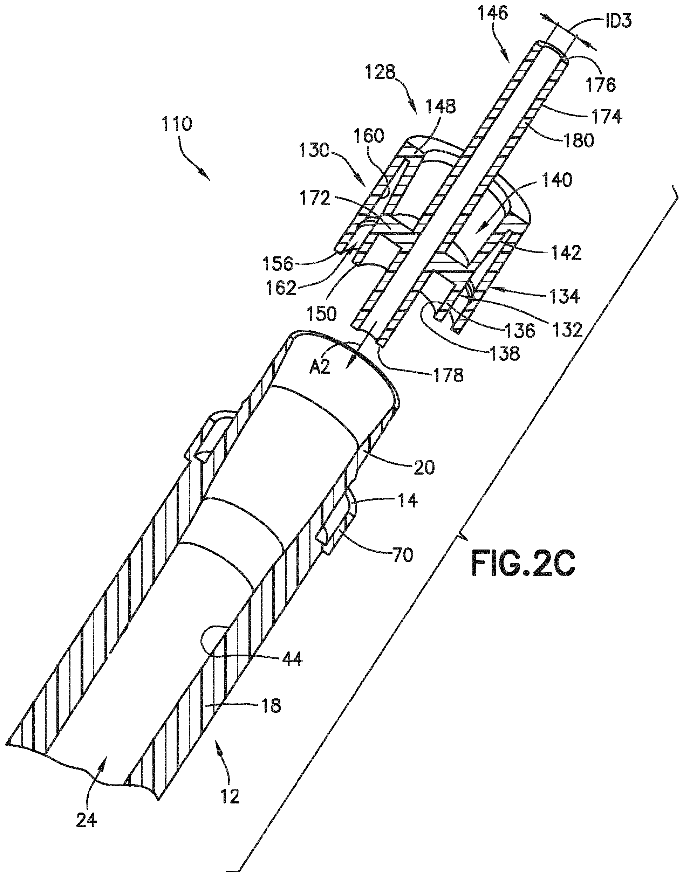

As shown most clearly in FIGS. 2B and 2C, the capillary rod or device can be a hollow elongated tube comprising an open top 176, an open bottom 178, and an annular sidewall 180 extending between the top 176 and the bottom 178. The sidewall 180 of the elongated tube can define a narrow flow channel extending from the top 176 to the bottom 178 of the rod sized for capillary action of the sample through the narrow flow channel.

As shown in FIGS. 2A-2C, the capillary action rod or device is mounted to the cover 130 by a wall 172 extending radially between an outer surface 174 of the capillary action rod or device and the inner surface 138 of the inner portion 132 of the cover 130, such that the rod or device extends through the channel 140 defined by the inner surface 138 of the inner portion 132 of the cover 130. The distal or top end 176 of the rod can extend distally beyond a top end 148 of the cover 130 in a distal direction. In a similar manner, a proximal or bottom end 178 of the rod can extend beyond the bottom end 150 of the cover 130. For example, the annular sidewall 180 of the rod or device can have a total length L3. Further, the sidewall 180 can extend distally beyond the top end 148 of the cover 130 by a distance D1. The rod can also extend proximally beyond the bottom end 150 of the cover 130 in a proximal direction by a distance D2.

The accessory 128 of FIGS. 2A-2C is mounted to the collection container 12 in a similar manner as in previous examples. Specifically, as shown in FIG. 2C, the accessory 128 is initially separate from the collection container 12. When ready to obtain the biological sample, the accessory 128 can be attached to the collection container 12 by inserting the inner portion 132 of the cover 130 through the open top end 14 of the collection container 12 in a direction of arrow A2 (shown in FIG. 2C). The cover 130 is then advanced onto the collection container 12 causing a bottom end 156 of the outer portion 134 to rest against the annular ridge 70 or ring of the collection container 12 and the open top end 14 of the collection container 12 to move into the annular slot 162 defined by the outer surface 142 of the inner portion 130 and the inner surface 160 of the outer portion 134 of the cover 130.

### Accessory for Blood Sample Collection and Puncture Device

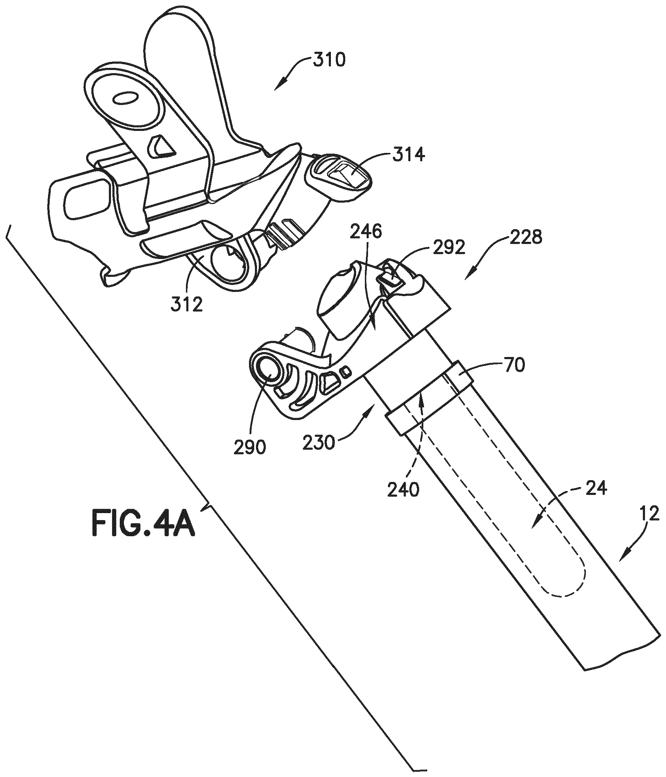

FIGS. 3A-4D show another example of a sample collection assembly 210 including an accessory 228 configured to be removably attached to the open top end 14 of the collection container 12. The accessory 228 is configured to be connected to a puncture device 310 (shown in FIGS. 4A-4D), also referred to as a blood collection device or blood collection assembly. The puncture device 310 includes a holding structure or holder for supporting a digit or another body part (e.g., a finger or toe of the patient) and includes or is coupled with a lancet for puncturing the skin surface of the patient. The puncture device 310 can also include various surfaces and flow channels for guiding the biological sample from the puncture location to, for example, the interior reservoir 24 of the collection container 12. The accessory 228 is shown in proximity to the collection container 12 in FIGS. 3A-3C. The collection container 12, accessory 228, and puncture device 310 are shown in FIGS. 4A-4D.

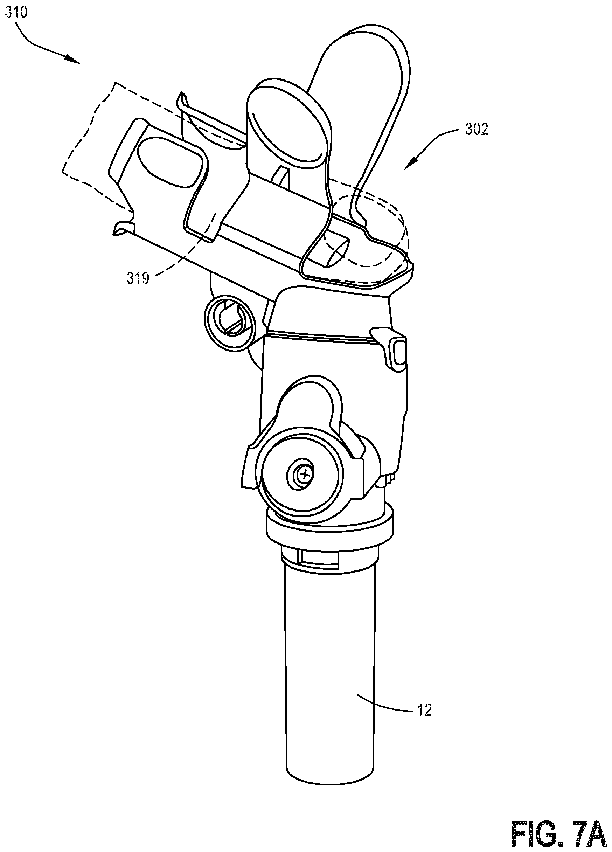

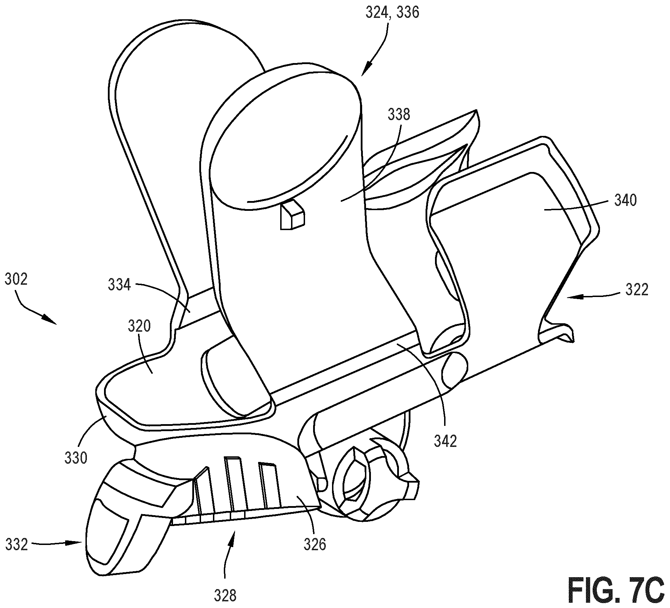

The accessory 228 can include one or more connectors, latches, locks, or similar mechanical fasteners for engaging the puncture device 310, thereby securing the puncture device 310 to the accessory 228. Once the puncture device 310 is secured to the collection container 12 and the patient's skin is punctured, the biological sample can flow through the guiding surfaces or channels of the puncture device 310, through the accessory 228, and into the interior reservoir 24 of the collection container 12. The puncture device 310 can be any of a variety of commercially available or specially made devices for puncturing a patient's skin and obtaining a fluid biological sample, including lancets, needles, sharps, or similar devices, as are known in the art, which can be adapted for engagement with the accessory 228 of the present disclosure. An exemplary puncture device 310, referred to as a capillary blood collector device, which can be used with the accessory of the present disclosure, is shown in FIGS. 4A-4D and FIGS. 7A-7E. Other puncture devices or blood collection devices that can also be adapted to be used with the accessory 228 are described in U.S. Patent No. 11,399,755, entitled "Device for Obtaining a Blood Sample"; U.S. Patent Appl. Pub. No. 2019/0223772, entitled "Device for the Attached Flow of Blood"; and PCT Appl. Pub. No. WO 2020/167746, entitled "Capillary collector with rotatable connection," the disclosure of each of which is incorporated herein by reference in its entirety.

As in previous examples, the accessory 228 of FIGS. 3A-4D comprises the cover 230 comprising the inner portion 232 configured for insertion into the collection container 12 and the outer portion 234 extending over the open top end 14 of the collection container 12. The inner portion 232 includes a sidewall 236 having an inner surface 238 defining a channel 240 through the accessory 228 and an outer surface 242 with an outer diameter sized to seal against an inner surface 44 of a sidewall 18 of the container 12.

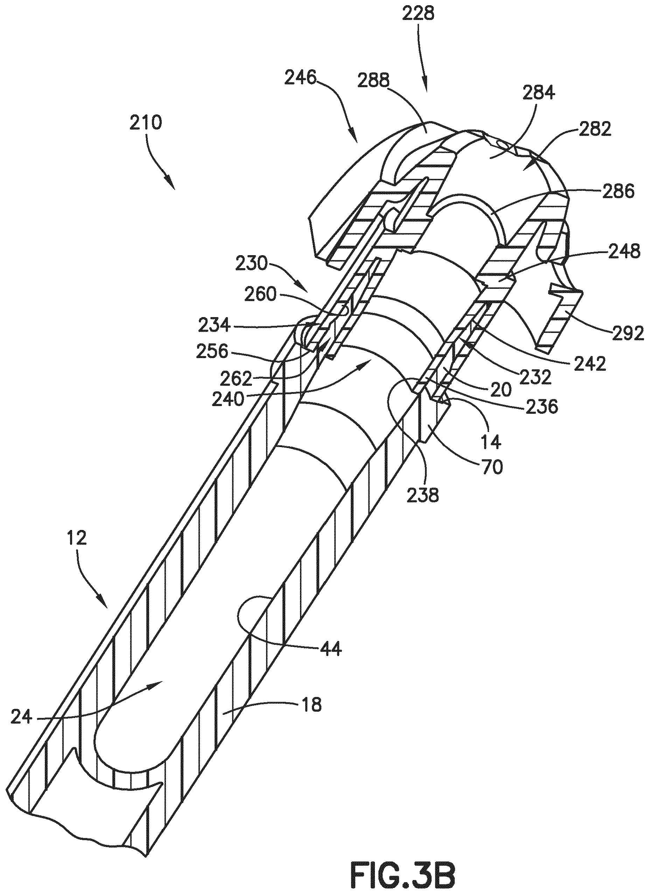

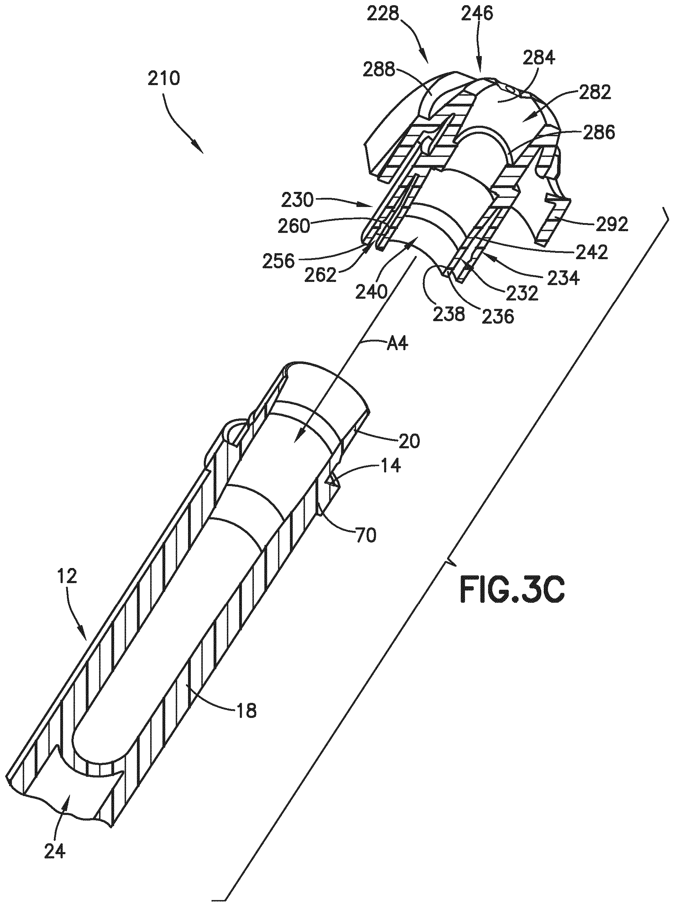

The accessory 228 also includes a collector 246 extending distally from the cover 230 configured for directing an obtained biological sample to the channel 240 defined by the inner surface 242 of the inner portion 232 of the cover 230. As shown in FIGS. 3A-3C, the collector 246 is an annular or tubular body defining a channel 282 configured to guide the biological sample to the channel 240 extending through the cover 230 or cover portion of the accessory 228. The tubular body can include a funnel-shaped or tapered inner surface 284 for directing the biological sample towards the channel 240 of the cover 230. In some examples, the collector 246 can also include an annular shelf 286, shoulder, ridge, or similar supporting surface on the inner surface 284 of the collector configured to align with and support a corresponding outflow port or outlet of the puncture device 310 so that the biological sample flows directly from the puncture device 310 into the channel 240 defined by the inner portion 232 of the cover 230. As shown most clearly in FIGS. 3B and 3C, the collector 246 can also include an exterior shelf 288 or shoulder portion on an exterior side of the collector 246 configured to receive corresponding structures of the puncture device 310 for supporting the puncture device 310 when it is mounted to the accessory 228.

The collector 246 of the accessory 228 can also include a connector 290 extending from a portion of the collector 246 configured to engage a corresponding receptor 312 (shown in FIGS. 4A-4D) of the puncture device 310 for securing the puncture device 310 to the accessory 228. When engaged to the connector 246, the puncture device 310 is positioned over the channel 240 defined by the inner portion 232 of the cover 230, meaning that the sample passes from the outlet or port of the puncture device 310 into the channel 240. The biological sample then passes through the channel 240 of the cover 230 into the interior reservoir 24 of the collection tube 12. In particular, the puncture device 310 and accessory 228 can be configured such that the obtained biological sample flows through the channel 282 of the collector 246 and the channel 240 of the cover 230 into the interior reservoir 24 of the collection container 12 by gravity.

In some examples, the connector 290 can be a post, protrusion, or protruding member, such as a cylindrical member, extending from the exterior surface of the collector 246. The protruding member can be configured to be received within and/or inserted into the corresponding receptor 312 (shown in FIGS. 4A-4D), such as an annular ring or circular opening, of the puncture device 310 for removably securing the puncture device 310 to the accessory 228. The connector 290 can include ribs, lips, protrusions, ridges, or similar raised portions extending about an outer surface of the connector 290, such that the connector 290 forms a snap fit connection with the receptor 312 of the puncture device 310.

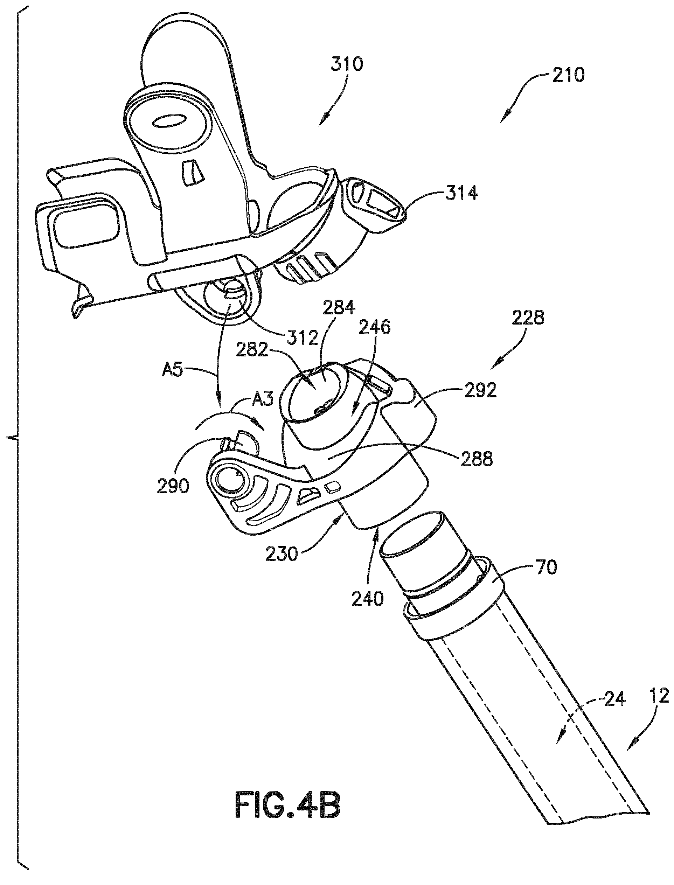

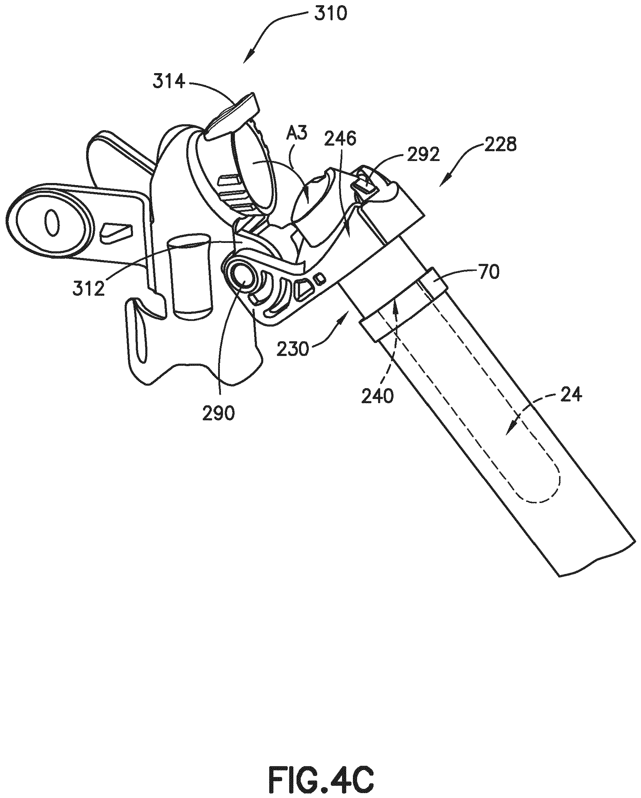

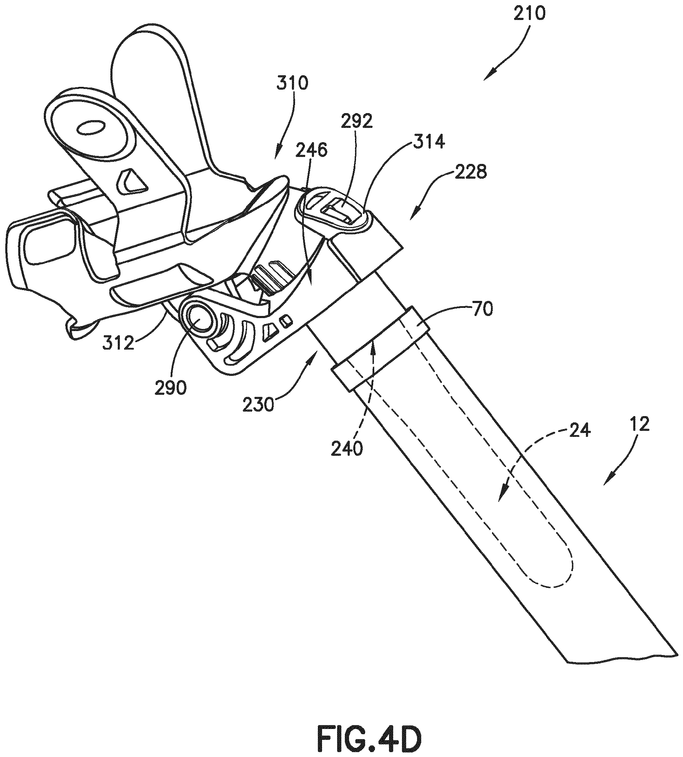

In some examples, the connector 290 is configured to rotatably engage the puncture device 310. For example, the connector 290 can be shaped to allow the receptor 312 of the puncture device 310 to rotate about the connector 290 in clockwise and/or counter-clockwise connections. The puncture device 310 can be configured to rotate in a direction of arrow A3 (shown in FIGS. 4B and 4C) from a pre-use position (shown in FIG. 4C), where an outflow port of the puncture device 310 is away from the channel 282 of the collector 246, to a use-position (shown in FIG. 4D), where the outflow port of the puncture device 310 is connected to or covers the opening to the channel 282 of the collector 246. For example, the practitioner may rotate the puncture device 310 towards the opening to the channel 282 when ready to perform a skin puncture and obtain the biological sample. The practitioner may rotate the puncture device 310 in an opposite direction, which moves the outflow port of the puncture device 310 away from the channel 282, in order to access the interior reservoir 24 of the collection container 12. Beneficially, the rotatable connection between the puncture device 310 and the collector 246 means that the practitioner can access the interior reservoir 24 of the collection container 12 without needing to disconnect the puncture device 310 from the accessory 228.

In some examples, the accessory 228 further comprises a latch 292 configured to engage a portion of the puncture device 310 for maintaining the puncture device 310 in the use position shown in FIG. 4D (i.e., the position where the biological sample passes from the puncture device 310 to the channel 282 defined by the collector 246 of the cover 230). For example, the latch 292 can be a protrusion, such as a square or rectangular shaped protrusion, extending from the exterior surface of the collector 246 on a side of the collector 246 opposite from the connector 290. As shown in FIGS. 4A-4D, the puncture device 310 includes a corresponding latch receptor 314, such as a square or rectangular ring or opening, configured to receive the latch 292 for locking the puncture device 310 in the use position.

The accessory 228 of FIGS. 3A-4D is mounted to the open top end 14 of the collection container 12 in the same was as in previous examples. Specifically, as shown in FIG. 3C, the accessory 228 can initially be provided separated from the collection container 12. When ready to collect a sample, the accessory 228 can be attached to the collection container 12 by inserting the inner portion 232 of the cover 230 through the open top end 14 of the collection container 12, as shown by arrow A4 in FIG. 3C. The cover 230 is then advanced onto the collection container 12 causing a bottom end 256 of the outer portion 234 to rest against the annular ridge 70 or ring of the collection container 12 and with the open top end 14 of the collection container 12 received within the annular slot 262 defined by the outer surface 242 of the inner portion 232 and the inner surface 260 of the outer portion 234 of the cover 230. Once the accessory 228 is mounted to the collection container 12, the puncture device 310 is mounted to the accessory 228 by inserting the connector 290 or protruding member of the accessory 228 through the receptor 312 of the puncture device 310, as shown by arrow A5 (shown in FIG. 4B), thereby removably engaging the puncture device 310 to the accessory 228. Once the puncture device 310 is attached to the accessory 228, the puncture device 310 can be moved from the pre-use position (shown in FIG. 4C) to the use position (shown in FIG. 4D) by rotating the receptor 312 about the connector 290, as shown by arrow A3 (in FIGS. 4B and 4C), thereby moving the puncture device 310 over the channel 282 of the collector 246 and causing the latch receptor 314 of the puncture device 310 to engage the latch 292 of the collector 246.

### Capillary Sample Collection Container

As previously described, the accessory devices or accessories 28, 128, 228 disclosed herein are configured to be connected to a variety of different shapes and configurations of collection containers including, but not limited to, the collection container 12 shown, for example, in FIGS. 1A-4D. The accessories 28, 128, 228 can also be connected to different types of collection containers including collection containers of varying shapes, lengths, and material compositions. Having described features of the exemplary accessories 28, 128, 228 in detail, additional features of the sample collection assembly 10 including the collection container 12 and cap 26 will now be described with reference to FIGS. 5A-5D. Specifically, these figures show the sample collection assembly 10 comprising the collection container 12, also referred to as a collection tube, for collecting biological sample, and the cap 26 configured to be mounted to the open top end 14 of the container 12 for sealing an obtained biological sample within the interior reservoir 24 of the collection container 12. For example, after a biological sample is obtained, the practitioner can remove the accessory 28, 128, 228 from the open top end 14 of the collection container 12 and attach the cap 26 to the open top end 14 of the container 12 for storing and/or preserving the biological sample contained therein. However, it is understood that components of the collection assembly 10 and collection container 12 shown in FIGS. 5A-5D are merely exemplary and that many other types and configurations of collection containers can also be used with the accessory devices or accessories 28, 128, 228 of the present disclosure.

As previously described and as shown in FIGS. 5A-5D, the collection container 12 includes a body or tube having the open top end 14, the second or bottom end 16, and the annular sidewall 18 extending therebetween. The open top end 14 can be a suitable size and/or shape for collecting samples from a puncture location, such as a skin surface of a patient. In some examples, the open top end 14 can have a flat, 360° lip feature 20 surrounding the open top end 14 of the container 12. When the bottom end 16 of the container 12 is open, the sample collection container 12 can also include a plug 22 configured to be coupled to the bottom end 16 of the collection container 12 to close the bottom end 16 of the collection container 12. The annular sidewall 18 and/or any interior walls of the collection container 12 can define the interior or interior reservoir 24 of the container 12 sized to contain a volume of a biological sample sufficient for a diagnostic test to be performed. For example, the interior reservoir 24 can have a volume of about 600 µL to about 1000 µL.

In some examples, the collection container 12 is a micro-tube suited for capillary collection of blood samples having overall exterior dimensions conforming to a standard tube (e.g., 13 mm x 90 mm) so as to be compatible with standard testing instruments and/or automation processes. The collection container 12 can be formed by a suitable molding process, such as injection molding from suitable plastic or composite material, such as a rigid or semi-rigid material (e.g., polypropylene). The collection container 12 can also include fill lines corresponding to a volume of the biological sample contained within the collection container 12.

In some examples, the collection containers 12 are configured to be compatible with most existing tube-type identification and recognition systems, such as tube-type identification and recognition systems comprising vision/camera systems. The vision/camera systems can be configured to read unique tube outer profiles, thereby recognizing and sorting the tubes appropriately for further analytical processing.

In some examples, the internal reservoir 24 of the collection container 12 can have an overall height-to-diameter ratio so as to create a taller column of blood or specimen within the collection container 12, even when the volume of blood or specimen collected is relatively low (e.g., 800 µL or less), as compared to a conventional container having an internal reservoir having a constant diameter and straight walls.

In some examples, the collection container 12 includes the open lip portion 20 that is uniform along its entire circumference meaning that the lip portion 20 does not include scoops, ridges, protrusions, or other extensions found in many conventional collection containers. Specifically, the open lip portion 20 can be substantially planar around an entire 360° circumference of the open top end 14 of the collection container 12. Further, the open lip portion 20 can be flat, such that a plane passing over the lip portion is transverse to the longitudinal axis X1 of the collection container 12. The uniform open lip portion 20 avoids disadvantages of conventional collection tubes, where extension or collector portions extending distally from the top of the container may not be compatible with automated processing machines. In particular, as previously described, the collection container 12 with the uniform lip portion 20 has the same appearance regardless of orientation. As such, the collection container 12 has an outer geometry that can be more easily identified by vision/camera systems regardless of how the collection container 12 is stored or oriented.

As shown in FIGS. 5A-5E, as well as in FIGS. 6A and 6B, the assembly 10 can also include a cap 26, which is configured to be attached to the collection container 12 to cover and seal the collection container 12 and any sample contained therein. The cap 26 can be removably attachable to the collection container 12 after collection of the sample contained therein and removal of the accessory 28, 128, 228 from the open top end 14 of the container. In some examples, the cap 26 can be made from a mixture of a rigid or semi-rigid material and a flexible material, such as a thermoplastic elastomer. Further, in some examples, the materials and/or geometry of the cap 26 can be selected to allow for one-handed closure and initial sealing of the collection container 12, meaning that the cap 26 can be closed easily with little required force. The cap 26 should also provide a high sealing pressure during centrifugation to prevent leaking. In some examples, the centrifugation process can be a reverse centrifugation process, in which the collection container 12 is inserted into the centrifuge in an upside-down orientation (i.e., with the lid of the collection container 12 positioned in the bottom of a receptacle receiving cavity or well of the centrifuge). Accordingly, the cap 26 can be designed to withstand forces exerted on the lid during reverse centrifuging. In some examples, a latch (not shown) is provided to secure the cap 26 in the closed position during storage, transport, and processing of the collection container 12.

In some examples, the capillary collection container assembly 10 further comprises the bottom plug 22. The bottom plug 22 can be configured to be coupled to the collection container 12 to effectively close the open bottom 16 of the container 12. In this way, collection container 12 has or defines a closed lower internal reservoir or bottom cavity 74 meaning that the collection container 12 can be used with existing automated racks and carriers. The bottom plug 22 can be configured for a press-fit interference connection to the collection tube 12 within open bottom end 16 of the collection container 12.

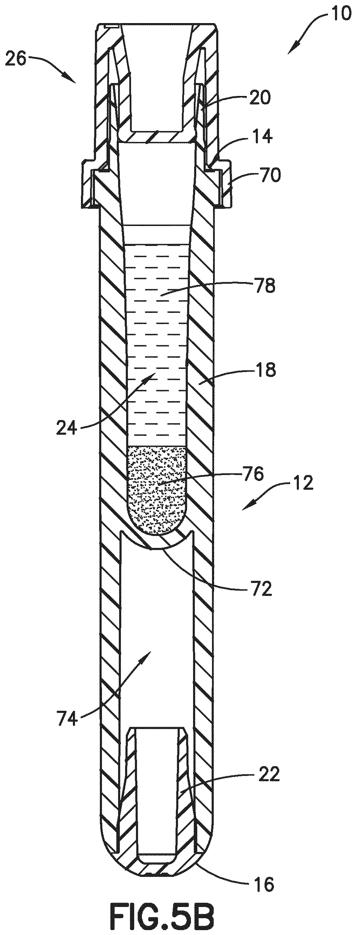

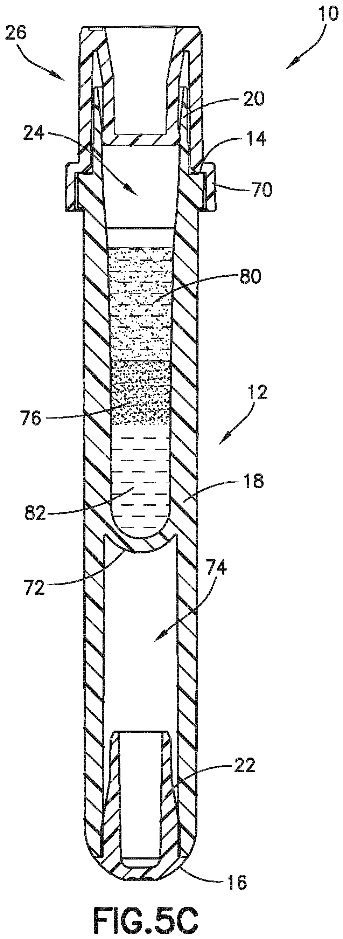

FIGS. 5B and 5C are cross-sectional views of the collection container assembly 10 in both a pre-centrifugation state (FIG. 5B) and a post-centrifugation state (FIG. 5C). As shown in FIGS. 5B and 5C, the cap 26 is configured to be coupled to the collection container 12 about the open top end 14 of the container 12 so as to effectively close and seal the internal reservoir 24. More specifically, in some examples, an upper portion of the collection container 12 can define the internal reservoir 24 for the collection, containment, and eventual transfer of biological specimens. The internal reservoir 24 can extend from the open top end 14 of the container 12 to a rounded bottom 72 of the collection container 12. The rounded bottom 72 can be an internal wall extending inwardly from the inner surface 44 of the sidewall 18 of the container 12, which defines a bottom portion of the internal reservoir 24. In some examples, the internal reservoir 24 can be coated with one or more additives (e.g., a clot activator, lithium heparin, a hemorepellancy additive, etc.). The additives can be sprayed or otherwise injected into the collection container 12 for preserving a blood or specimen sample contained within the collection container 12 during storage or for other diagnostic purposes as is known by those of ordinary skill in the art.

As shown in FIGS. 5B and 5C, the internal reservoir 24 is defined within the sidewall 18 of the container 12, with sidewall 18 having a generally angled and rounded profile terminating in the rounded bottom 72. The angled and rounded profile of internal reservoir 24 can be configured to allow for an overall increased height-to-diameter ratio so as to create a taller column of blood or specimen within the collection container 16, thereby providing for better visualization and/or simplified collection of the specimen collected within the internal reservoir 24 and reduced "dead volume", compatibility with sample volume detection systems compared to containers having a reservoir extending entirely from the top 14 to the bottom 16 and defined by the inner surface 44 of the sidewall 28 of the container 12. For example, the inner surface 44 can be angled between 2.5° to 10° relative to the longitudinal axis X1 of the collection container 12, thereby providing for a taller specimen column as compared to a tube having an internal reservoir whose sidewalls are not angled (or minimally angled).

In some examples, a lower portion of the collection container 12 includes a generally hollow or "false" bottom cavity 74 defined by the sidewall 18 of the container 12, the bottom 16 of the container 12, and the rounded bottom 72 of the collection container 12. The "false" bottom cavity 74 of the lower portion of the collection container 12 can assist in injection molding of the collection container 12 by promoting plastic flow. Furthermore, by extending the lower portion of the collection container 12 in this way, and also by providing bottom plug 22 in the bottom end 16 of the container 12, the collection assembly 10 can be more readily compatible with standard medical testing instruments and/or automation processes.

As noted above, FIG. 5B portrays the collection container 12 in the pre-centrifugation state. In such a state, the internal reservoir 24 can contain a gel separator substance 76 at a bottom portion thereof, and a specimen sample 78 (e.g., a blood sample) having an overall lower density than the gel separator substance 76 is maintained above the gel separator substance 76. Upon centrifugation of such, the collection container 12, primary components of the blood (e.g., the plasma/serum and the hematocrit comprised primarily of red blood cells) separate by density, with the denser hematocrit settling at the bottom of the internal reservoir 24, and the less dense plasma/serum collecting above the hematocrit. A gel separator substance 76, meanwhile, is configured to have a density between that of the plasma/serum and hematocrit. Accordingly, upon centrifugation, the gel separator substance 76 forms a barrier between the plasma/serum and the hematocrit.

FIG. 5C shows the collection container 12 in a post-centrifugation state. In FIG. 5C, the sample specimen 78 has separated into two primary component parts, specifically into a plasma/serum portion 80 and a hematocrit portion 82. Also, after centrifugation, the gel separator substance 76 migrates from the bottom portion of the internal reservoir 24 to create an effective barrier between the plasma/serum portion 80 and the hematocrit portion 82. Due to the overall reduced size of the internal reservoir 24 in relation to the size of the entire collection container 12, the plasma/serum portion 80 of the specimen sample 78 is easily visible to the user and accessible for analyzer/probe aspiration, even when the volume of collected capillary blood is relatively low.

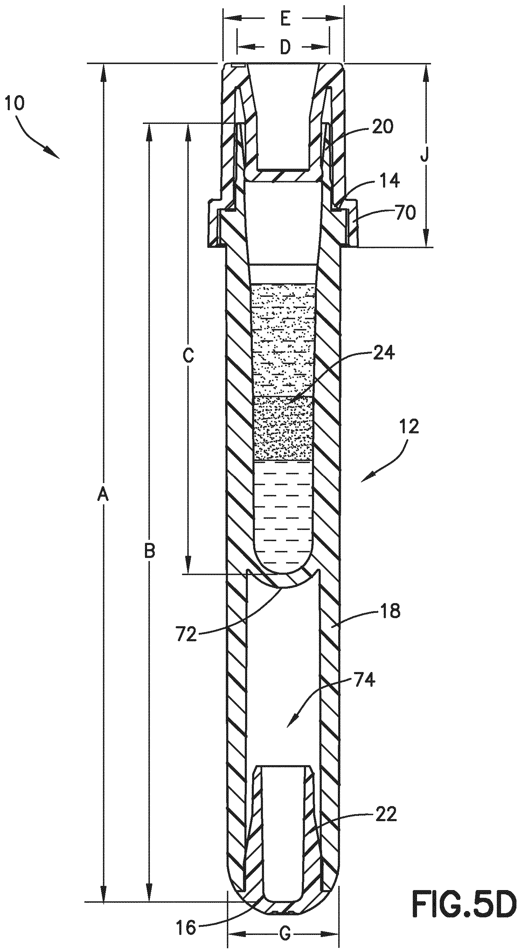

FIG. 5D and Table 1 show dimensions of the collection container 12 and cap 26 of the collection container assembly 10. It is to be understood, however, that the dimensions shown in FIG. 5D and Table 1 and described herein are merely illustrative and that the collection container assembly 10 is not limited as to such dimensions or dimension ranges.

**TABLE 1:**

| **Dimension Identifier** | **Part Features** | **Nominal Dimensions (mm)** |
|---|---|---|
| A | Tube Length w/ cap | 75-95 |
| B | Tube Length w/o cap | 70-90 |
| C | Tube Interior Reservoir Length | 35-55 |
| D | Tube Opening | 5-12 |
| E | Tube Opening | 5-12 |
| (Not shown) | Tube Bottom | 3-9 |
| G | Tube Bottom | 7-15 |
| (Not shown) | Tube | 10-15 |
| J | Cap Height | 15-25 |

As is evident from the dimensions shown and described with respect to FIG. 5D and Table 1, the overall length of interior reservoir 24 (C) is far smaller than the overall length of the collection container 12 (B), thereby enabling the collection container 12 to handle a small volume specimen, yet maintain a conventional form factor for use with standard medical testing instruments and/or automation processes. Furthermore, as the collection container bottom inner diameter is significantly smaller than the collection container opening inner diameter (D), resulting in the tapered sidewall 18, a taller column of blood or specimen is provided, allowing for improved visibility of the specimen, specimen collection, and/or barrier separation between specimen components.

FIGS. 6A and 6B are enlarged drawings of the cap 26, which can be connected to the open top end 14 of the collection container 12 after the accessory 28 is removed from the collection container 12. The cap 26 is configured to be substantially cylindrical in shape, with a bottom annular portion 84 defining an opening 86, as well as a top annular surface 88. Extending between the top annular surface 88 and the bottom annular portion 84 are a plurality of vertically-extending ribs 90. The ribs 90 may extend 360° around the cap 26 so as to provide for easy gripping and de-capping during automated handling, when compared with conventional caps having a generally cylindrical shape and/or a rib placement that extends less than 360° around the cap. Interior to the top annular surface 88, the cap 26 may include a cavity portion 92 (shown in FIG. 6B) defined by a sidewall. The cavity portion 92 and sidewall may be sized and configured so as to fit within an inner diameter of the open top end 14 of the collection container 12, thereby effectively closing/sealing the open top end 14 when the cap 26 is provided on the collection container 12.

Furthermore, adjacent to the bottom annular portion 84, the cap 26 may further include a downwardly extending sidewall portion 94 (shown in FIG. 6B), which creates a gap between the bottom annular portion 84 and the sidewall portion 94. While not shown, the cap 26 may be held in place on the collection container 12 via a snap groove on the cap 26 and/or the collection container 12, with the snap groove providing audible and/or tactile feedback to the user to confirm that the cap 26 is secured to the collection container 12. Furthermore, a pedestal portion of the cap 26 may effectively seal the contents of the interior reservoir 24 within the collection container 12. While the interface between cap 26 and collection container 12 is described herein as a snap fit, it is to be understood that cap 26 and/or collection container 12 may be coupled by any appropriate method such as, e.g., a threaded interface, a bayonet interface, etc.

### Sample Collection Assembly and Puncture Device

As previously described, the accessory 228 of FIGS. 3A-4D can be configured to engage with a variety of different types of puncture devices or sample collection devices, such as devices comprising lancets, sharps, needles, and sharpened elongated members for skin puncture. Additional features of an exemplary puncture device 310 that can be used with the accessory 228 will now be described in detail with reference to FIGS. 7A-7E. However, it is understood that other types of puncture devices and sample collection devices can also be used with the accessories of the present disclosure. For example, certain puncture devices and sample collection devices can be modified to include various connectors, receptors, latches, and other mechanisms for mounting the device to the previously described accessories 228 and similar devices.

As shown in FIGS. 7A-7E, the blood collection devices 310 can be, for example, a self-contained and fully integrated finger-based capillary blood collection device with the ability to lance, collect, and stabilize a high volume capillary blood sample, e.g., up to or above 500 microliters, in the sample collection container 12. The puncture device 310 can also be formed from separable components (e.g., a finger cuff and lance) that can be connected and/or used together to obtain a blood sample.

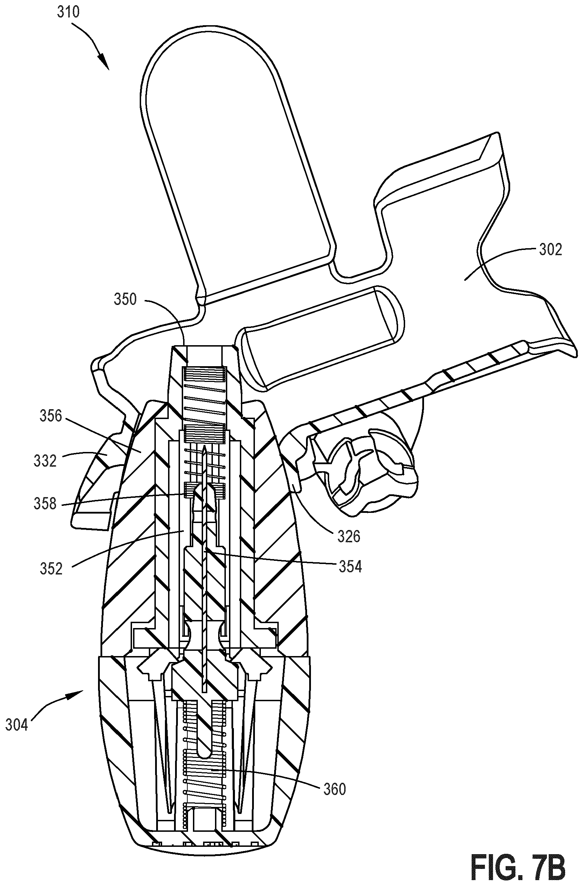

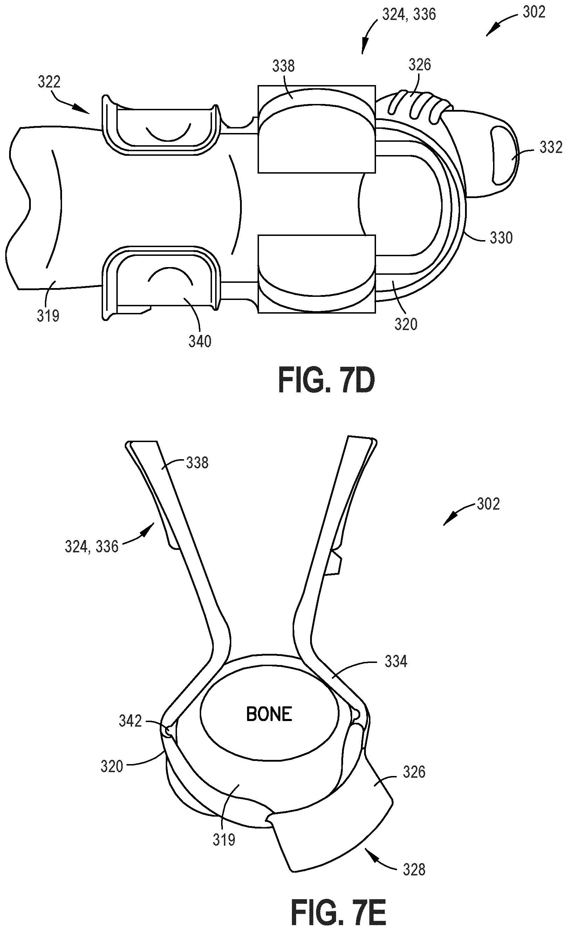

With reference to FIGS. 7A and 7B, the puncture device 310 includes an integrated holder 302 and a lancet housing or lancet 304 (shown in FIG. 7B) for puncturing a finger 319 (shown in FIGS. 7A, 7D, and 7E) of the patient. In other examples, the lancet housing 304 can be separate from the holder 12. The holder 302 is configured to receive a sample source, e.g., the finger 19 of a patient, for supplying a biological sample, such as a blood sample. The holder 302 generally includes a finger receiving portion 320 having a first opening 322, an actuation portion 324, a port 326 having a second opening 328, and a finger end guard 330. In some examples, the finger end guard 330 provides a stop portion for properly aligning and securing the finger 319 within the holder 302. The finger end guard 330 further assists in ensuring the patient's finger 319 is placed at a proper position within the finger receiving portion 320 so that applied pressure to the patient's finger 319 will result in adequate blood flow. The finger end guard 330 can have a curved fingertip rest that ensures the patient's finger 319 stops at an end of the finger receiving portion 320, while permitting the patient's finger nail to clear the end of the finger receiving portion 320. The finger receiving portion 320 permits use of the holder 312 with artificial and natural fingernail styles present in the patient population.

The first opening 322 of the finger receiving portion 320 is configured for receiving the sample source, e.g., the finger 319. The sample source may also include other parts of the body capable of fitting within the first opening 322, such as toes or other extremities. The port 326 is in communication with the finger receiving portion 320. For example, with a finger 319 received within the holder 302, the port 326 is in communication with a portion of the finger 319. The second opening 328 of the port 326 is configured for receiving the lancet housing or lancet 304 (shown in FIG. 7B) and for receiving the collector 246 of the accessory 228 for establishing fluid communication between the port 326 and the internal reservoir 24 of the collection container 12. In some examples, the port 326 further includes a locking portion 332 for securely receiving the lancet housing or lancet 304 and the collector 246 of the accessory 228 within the port 326.

The actuation portion 324 of the puncture device 310 is transitionable between a first position, in which the holder 302 defines a first diameter, and a second position, in which the holder 302 defines a second diameter, with the second diameter being less than the first diameter. Further, in the first position, the holder 302 defines a first elliptical shape. In the second position, the holder 302 defines a second elliptical shape, with the first elliptical shape being different than the second elliptical shape. In this manner, with the holder 302 in the second position with a reduced diameter, a portion of the holder 302 contacts the sample source (e.g., the finger 319) and the actuation portion 324 of the holder 302 is able to pump and/or extract blood.

In some examples, the actuation portion 324 includes a contact member 334. With the actuation portion 324 in the first position, the contact member 334 is in a disengaged position, i.e., the contact member 334 is provided in a first position with respect to the sample source, such that the contact member 334 may be in slight contact therewith. With the actuation portion 324 in the second position, the contact member 334 is in an engaged position, i.e., the contact member 334 is provided in a second position with respect to the finger 319, such that the contact member 334 is in an applied pressure contact with the finger 319, and the actuation portion 324 of the holder 302 is able to pump and/or extract blood. For example, with the contact member 334 in the engaged position, the contact member 334 exerts a pressure on the sample source.

In some examples, the actuation portion 324 includes a pumping member 336 for applying pressure to the finger 319, such as a pair of opposed tabs or wings 338. Each wing 338 can include a contact member 334. The holder 302 can also include a living hinge portion 342. The living hinge portion 342 allows the practitioner or patient to squeeze the wings 338 between a first position (passive state) and a second position (active state). It is believed that use of the tabs or wings 338 to draw blood out of a patient's finger 319 minimizes hemolysis while maintaining an adequate flow of blood from the patient's finger 319. A resting position and hinge of the wings 338 are designed to maintain contact and retention with the smallest patient finger that can fit into a holder 302, while flexing to accommodate the largest patient fingers within a holder 302 without blood occlusion. In some examples, the wings 338 may be positioned on the finger receiving portion 320 at a position located proximal of a patient's fingernail and distal of a patient's first knuckle to avoid hard tissues on the patient's finger 319.

The holder 302 can be configured to allow a user to repeatedly squeeze and release the wings 338 to pump and/or extract blood from a finger 319 until a desired amount of blood flows through the accessory 228 and into the collection container 12. The wings 338 are configured to flex to maintain gentle contact with a range of patient finger sizes that may be used with the holder 302 and to retain the holder 302 on the patient's finger 319. The wings 338 may also provide active pressure features for the holder 302.

In some examples, the holder 302 can include a stability extension portion 340. This provides additional support for the holder 302 to be securely placed onto the finger 319. In one example, the finger receiving portion 320 forms a generally C-shaped member and includes a plurality of inner gripping members for providing additional grip and support for the holder 302 to be securely placed onto a patient's finger 319. The stability extension portion 340 assists in maintaining contact with the patient's finger 319 during use of the holder 302 while avoiding the blood supply and knuckles of the patient's finger 319.

The puncture device 310 for obtaining the blood sample also includes the lancet housing or lancet 304 (shown in FIG. 7B) that is removably connectable to the port 326 of the holder 302 prior to inserting the port into the tubular body of the accessory. The lancet housing or lancet 304 can include an inlet or opening 350, an interior 352, a puncturing element 354, an engagement portion 356, a retractable mechanism 358, and a drive spring 360. The puncturing element 354 can be moveable between a pre-actuated position, wherein the puncturing element 354 is retained within the interior 352 of the lancet 304 or lancet housing, and a puncturing position, wherein at least a portion of the puncturing element 354 extends through the inlet 350 of the lancet housing or lancet 304 to lance a portion of a finger 319. In one example, the lancet 304 is a contact activated lancet and may be constructed in accordance with the features disclosed in U.S. Patent No. 9,380,975, entitled "Contact Activated Lancet Device", which is incorporated herein by reference in its entirety.

In some examples, the holder 302 and the lancet housing or lancet 304 are separate components that can be removably connectable to the port 326 of the holder 302. In such examples, the lancet housing or lancet 304 includes the engagement portion 356. The lancet housing or lancet 304 can be pushed into the port 326 of the holder 302, such that the engagement portion 356 of the lancet housing or lancet 304 is locked within the locking portion 332 of the holder 302. In this manner, the lancet housing 304 is securely connected and locked to the holder 302, such that the puncturing element 354 of the lancet housing 304 can be activated to lance or puncture a sample source, such as the finger 319. In some examples, the port 326 of the holder 302 includes a plurality of ribs for securing and locking the lancet 304 in the port 326. The ribs can also be positioned to assist in securing the collector 246 of the cover 230 within the port 326 when the puncture device 310 is mounted to the accessory 228. To activate the lancet 304, the lancet 304 is pushed against the finger 319 to activate the retractable mechanism 358 and drive spring 360 of the lancet 304 to lance the finger 319. After puncturing, the puncturing element 354 is immediately retracted and safely secured within the interior 352 of the lancet housing 304.

In order to use the puncture device 310 shown in FIGS. 7A-7E, a desired finger 319 is first cleaned and a holder 302 having an appropriate size for the desired finger 319 is selected and placed onto the finger 319 securely. Next, the lancet housing or lancet 304 is connected to the port 326 of the holder 312. As discussed previously, the lancet housing or lancet 314 is pushed into the port 326 of the holder 302, such that the engagement portion 356 of the lancet housing or lancet 304 is locked within the locking portion 332 of the holder 302. In this manner, the lancet housing or lancet 304 is securely connected and locked to the holder 302, such that the puncturing element 354 of the lancet housing 304 can be activated to lance or puncture the finger 319. With the lancet 304 connected to the port 326 of the holder 302, the lancet 304 is in communication with the finger 319.

When it is desired to activate the lancet 304 to lance the skin of the finger 319, the lancet 304 is pushed against a finger 319 to activate a retractable mechanism 358 of the lancet 304 to lance the finger 319. After the finger 319 is lanced to create blood flow from the finger 319, the lancet 304 is removed from the holder 302 and the puncture device 310 is engaged to the accessory 228 with the port 326 inserted into the collector 246 of the accessory 228 as previously described. With the collection container 12 properly secured to the puncture device 310 through the accessory 228, the patient or practitioner repeatedly squeezes and releases the wings 338 of the holder 302 to pump and/or extract blood from the finger 319 until a desired amount of blood is collected in the collection container 12. Advantageously, with the holder 302 placed onto a finger 319, the holder 302 does not constrict the blood flow and defines lancing and finger squeezing locations. The squeezing tabs or wings 338 provide a pre-defined range of squeezing pressure that is consistently applied throughout a finger 319. By doing so, the holder 302 provides a gentle controlled finger 319 massage that stimulates blood extraction and minimizes any potential hemolysis.

Once a desired amount of blood is collected within the collection container 12, the accessory 228 and puncture device 310 can be disconnected from the collection container 12. The collection container 12 can then be sealed via the cap 26 in order to provide a sealed sample that can be stored for a period of time and/or transported to another clinical facility, such as a laboratory, for testing and analysis.

### Blood Collection and Automated Processing Methods

Biological samples obtained using the collection containers 12, accessories 28, 128, 228 and assemblies 10, 110, 210 described herein can be processed by automated processing machines. In particular, as previously described, the collection containers 12 or tubes can include an open top end 14 that is flat, without protruding structures or other members, meaning that the containers 12 and tubes are adapted to work with a wide variety of automated processes, machines and devices known in the art. For example, the collection containers 12 can be manipulated by automated "pick-and-place" gripping and/or de-capping processes. Automated testing processes can also include using sorting machines to sort collection containers (e.g., by type, size, and/or contents). Other automated processes can include filling selected specimen collection containers with specific additives, centrifugation, vision-based specimen quality analysis, serum level analysis, decapping, aliquoting, and/or automated labeling of containers.

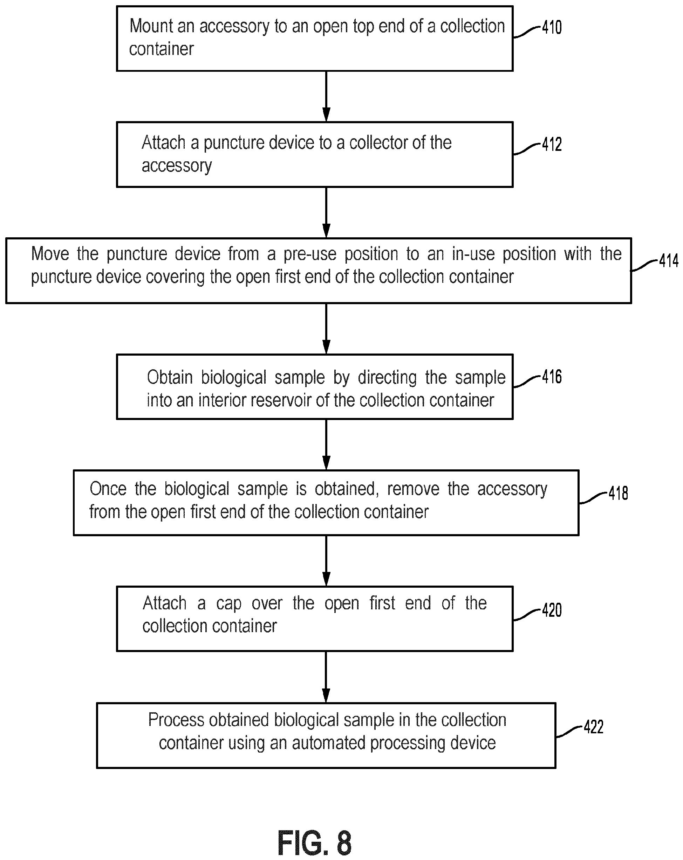

FIG. 8 is a flow chart showing a method for collecting a biological sample, such as a blood sample, using the collection container 12 and accessory 28, 128, 228 of the present disclosure and for automated processing of the collected biological sample contained in the interior reservoir 24 of the collection container 12. As shown in FIG. 8, the method includes, at step 410, mounting the accessory 28, 128, 228 to the collection container 12 by inserting an inner portion 32, 132, 232 of the accessory 28, 128, 228 through the open first end 14 of the collection container 12.

For an accessory 228 configured to be connected to a puncture device 310, the method can also include, at step 412, after mounting the accessory 228 to the collection container 12, attaching the puncture device 310 to a collector 246 of the accessory 228 by inserting the connector 290 of the collector 246 through a corresponding receptor 312 of the puncture device 310. At step 414, the method also can include moving the puncture device 310 from a pre-use position (shown in FIG. 4C), in which the puncture device 310 is spaced apart from the open first end 14 of the collection container 12, to an in-use position (shown in FIG. 4D), in which the puncture device 310 covers the open first end 14 of the collection container 12.

At step 416, the method further includes obtaining the blood sample by directing the blood sample through the collector 46, 146, 246 of the accessory 28, 128, 228 and into an interior reservoir 24 of the collection container 12 through a channel 40, 140, 240 defined by the inner portion 32, 132, 232 of the accessory 28, 128, 228. For example, obtaining a blood sample, such as a capillary blood sample, can include pricking a skin surface of the patient and drawing the blood sample into the interior reservoir 24 of the collection container 12 with the collector 46, 146, 246 of the accessory 28, 128, 228. For the accessory 228 that attaches to the puncture device 310, obtaining the blood sample can include inserting the patient's finger 319 into the holder 302 and puncturing the finger 319 with the lancet 304, as previously described.

At step 418, the method further includes, once the biological or blood sample is obtained, removing the accessory 28, 128, 228 from the open first end 14 of the collection container 12. For example, removing the accessory 28, 128, 228 can include grasping the accessory 28, 128, 228, such as between a thumb and forefinger, and pulling the accessory 28, 128, 228 away from the open top end 14 of the collection container 12, which causes the inner portion 32, 132, 232 of the accessory 28, 128, 228 to slide out of the interior reservoir 24 through the open top end 14 of the collection container 12. Optionally, at step 420, the method can further include after removing the accessory 28, 128, 228 from the collection container 12 and prior to processing the biological or blood sample, attaching a cap 26 over the open first end 14 of the collection container 12 to seal the biological or blood sample within the interior reservoir 24 of the collection container 12.

At step 422, the method further includes processing the obtained biological or blood sample in the collection container 12 using an automated processing device. For example, as previously described, the automated processing device can include one or more devices that perform one or more of the following automated testing processes: sorting collection containers 12 containing blood samples by type, size, and/or contents with an automated sorting machine; filling selected specimen collection containers 12 with specific additives; centrifugation; vision-based specimen quality analysis; serum level analysis; decapping; aliquoting; and/or automated labeling of collection containers 12.

While different examples of the accessories, collection containers, blood collection devices, and associated assemblies are shown in the accompanying figures and described hereinabove in detail, other examples will be apparent to, and readily made by, those skilled in the art without departing from the scope and spirit of the invention. Accordingly, the foregoing description is intended to be illustrative rather than restrictive. The invention described hereinabove is defined by the appended claims and all changes to the invention that fall within the meaning and the range of equivalency of the claims are to be embraced within their scope.

## Claims

1. A sample collection accessory device configured for attachment to an open end of a collection container, the accessory device comprising:
a cover comprising (i) an inner portion configured for insertion into the collection container comprising a sidewall having an inner surface defining a channel through the accessory device and an outer surface with an outer diameter sized to seal against an inner surface of a sidewall of the container and (ii) an outer portion extending radially outward from the inner portion configured to extend over an end of the sidewall of the collection container; and
a collector extending distally from the cover configured for directing a biological sample to the channel defined by the inner surface of the inner portion of the cover.

2. The accessory device of claim 1, wherein the cover and/or collector comprise a flexible plastic material comprising one or more of silicone, polypropylene, low-density polyethylene, synthetic rubber, or natural rubber.

3. The accessory device of claim 2, wherein the flexible plastic material comprises additives for increasing wettability of surfaces of the accessory device.

4. The accessory device of claim 1, wherein the inner portion of the cover is tapered, with an outer diameter of a first end of the inner portion being wider than an outer diameter of a second end of the inner portion, and wherein the second end of the inner portion is configured to be inserted into the collection container.

5. The accessory device of claim 1, wherein the outer portion comprises a first end connected to a first end of the inner portion, a second end, and an annular sidewall extending between the first end and the second end of the outer portion, and
wherein an outer surface of the inner portion and an inner surface of the sidewall of the outer portion define an annular slot sized to receive a lip feature extending from the open end of the collection container.

6. The accessory device of claim 1, wherein the accessory device and collection container are configured for collection and storage of a capillary blood sample.

7. The accessory device of claim 1, wherein the collector comprises at least one of a scoop, a funnel, or a capillary action member.

8. The accessory device of claim 1, wherein the cover and the collector are an integral part formed by a single molding process, such as injection molding.

9. The accessory device of claim 1, wherein the collector comprises a scoop comprising (i) a first portion connected to and extending from the inner portion of the cover for guiding the sample toward the inner portion of the cover, (ii) a second portion opposite the first portion, and (iii) a guiding surface extending between the first portion and the second portion.

10. The accessory device of claim 9, wherein the second portion of the scoop comprises a curved top edge, which curves in at least two dimensions.

11. The accessory device of claim 1, wherein the collector comprises a capillary action rod mounted to the cover by a wall extending radially between an outer surface of the capillary action rod and the inner surface of the inner portion of the cover.

12. The accessory device of claim 11, wherein the rod extends through the channel defined by the inner surface of the inner portion of the cover, and wherein the rod is hollow comprising an open first end, an open second end, and an annular sidewall extending between the first end and the second end.

13. The accessory device of claim 1, wherein the collector comprises a tubular body defining a channel configured to guide the sample to the channel of the cover and a connector on an exterior portion of the collector configured to engage a corresponding connector of a puncture device for removably connecting the puncture device to the accessory device.

14. The accessory device of claim 13, wherein, when engaged to the connector on the exterior portion of the collector, the puncture device is positioned over the channel defined by the tubular body for directing the biological sample through the tubular body and cover into an interior reservoir of the collection container.

15. The accessory device of claim 14, wherein the biological sample moves from the puncture device through the tubular body of the connector and cover into the interior reservoir of the collection container by gravity.

16. The accessory device of claim 13, wherein the connector is configured to rotatably engage the puncture device, such that the puncture device is movable from a pre-use position, where the puncture device is spaced apart from the open end of the collection container, to an in-use position, wherein the puncture device covers the open end of the collection container.

17. The accessory device of claim 13, wherein the connector comprises a cylindrical member configured for insertion into a corresponding receptor of the puncture device, and
wherein the cylindrical member is retained within the receptor of the sample collection container by a snap fit connection.

18. The accessory device of claim 17, wherein the collector further comprises a latch configured to engage a portion of the puncture device for maintaining the puncture device in an in-use position with the puncture device covering the open end of the collection container.

19. A collection assembly comprising:
a collection container comprising an open first end, a second end, and an annular sidewall extending between the first end and the second end, the annular sidewall of the collection container defining an interior reservoir configured to receive a biological sample; and
an accessory device removably mounted to the open first end of the collection container, the accessory device comprising:
a cover comprising (i) an inner portion inserted into the collection container comprising a sidewall having an inner surface defining a channel through the accessory device and an outer surface with an outer diameter sized to seal against an inner surface of the annular sidewall of the collection container and (ii) an outer portion extending radially outward from the inner portion and over the open first end of the collection container; and
a collector extending distally from the cover configured for directing the biological sample through the channel defined by the inner surface of the inner portion of the cover and into the interior reservoir of the collection container.

20. A method for automated blood sample processing, comprising:
mounting an accessory device to a collection container by inserting an inner portion of the accessory device though an open first end of the collection container, wherein the accessory device comprises:
a cover comprising (i) the inner portion comprising a sidewall having an inner surface defining a channel through the accessory device and an outer surface with an outer diameter sized to seal against an inner surface of a sidewall of the collection container and (ii) an outer portion extending radially outward from the inner portion configured to extend over the open first end of the collection container; and
a collector extending distally from the cover configured for directing the blood sample to the channel defined by the inner surface of the inner portion of the cover;
obtaining the blood sample by directing the blood sample through the collector and into an interior reservoir of the collection container through the channel defined by the inner portion of the accessory device;
once the blood sample is obtained, removing the accessory device from the open first end of the collection container; and
processing the obtained blood sample in the collection container using at least one automated processing device.
